# EUROPEAN PATENT APPLICATION

(11) **EP 3 913 362 A1**
(43) Date of publication of application: **24.11.2021**
(21) Application number: 20741628.0
(22) Date of filing: 14.01.2020
(51) Int. Cl.: G01N 30/88, B01D 15/00, B01J 20/08, B01J 20/10, B01J 20/20, B01J 20/282, B01J 20/283, B01J 20/284, B01J 20/30, B01J 20/34, G01N 30/26, G01N 30/46

(54) **METHOD FOR FRACTIONATING DIOXIN-TYPE COMPOUNDS**

(30) Priority: 18.01.2019 JP 2019006673
(71) Applicant: MIURA CO., LTD., Matsuyama-shi Ehime 799-2696 (JP)
(72) Inventor: FUJITA, Hiroyuki, Matsuyama-shi Ehime 799-2696 (JP); SUGA, Koichi, Matsuyama-shi Ehime 799-2696 (JP)
(74) Representative: Elkington and Fife LLP
(86) International application number: PCT/JP2020/000863
(87) International publication number: WO 2020/149255

(57) **Abstract**

In a standing pipe body (210), an adsorbent layer (240) filled with active magnesium silicate as an adsorbent and an alumina layer (250) positioned therebelow are arranged. A sample solution containing dioxins is applied into the pipe body (210) from the top, and an aliphatic hydrocarbon solvent is subsequently supplied into the pipe body (210) from the top. The aliphatic hydrocarbon solvent having dissolved dioxins in the sample solution passes through the adsorbent layer (240) and the alumina layer (250) in this order, and is discharged from a bottom of the pipe body (210). At this point, a dioxin group including non-ortho PCBs, PCDDs, and PCDFs is selectively trapped by the adsorbent layer (240), and mono-ortho PCBs are selectively trapped by the alumina layer (250).

## Description

### TECHNICAL FIELD

The present invention relates to the method for fractionating dioxins, and specifically relates to the method for fractionating dioxins contained in an aliphatic hydrocarbon solvent solution with the dioxins. The present application claims a priority based on Japanese Application No. 2019-006673 filed in Japan on January 18, 2019, and the contents of which are incorporated herein by reference.

### BACKGROUND ART

With concern over environmental contamination due to dioxins as highly-toxic substances, analysis and evaluation of a contamination status due to the dioxins have been, in each country, demanded for exhaust gas from a waste incineration facility, ambient air, water such as industrial waste or river water, fly ash generated at a waste incineration facility, soil and the like. For food, similar analysis and evaluation have been demanded in many cases.

The dioxins are generally a collective term of polychlorinated dibenzo-p-dioxins (PCDDs), polychlorinated dibenzofurans (PCDFs), and dioxin-like polychlorinated biphenyls (DL-PCBs). Of 209 isomer types of polychlorinated biphenyls (PCBs), the DL-PCBs are PCBs having toxicity similar to those of the PCDDs and the PCDFs, and include non-ortho PCBs and mono-ortho PCBs.

For evaluating contamination of a sample such as an environmental sample including ambient air, soil and the like or a food sample due to the dioxins, the dioxins need to be first extracted from the sample. In a case where the sample is a solid object such as soil or dry food, the dioxins are extracted from the solid object by, e.g., a Soxhlet extraction method. For example, in a case where the sample is liquid such as waste water or drinking water, the dioxins in the liquid are trapped and collected using a collector such as a filter, and thereafter, the collector is rinsed or the Soxhlet extraction method is applied to the collector to extract the dioxins collected by the collector. The dioxin obtained as described above is, quantitatively analyzed using a gas chromatograph mass spectrometry (GC/MS).

The extracts with dioxins contain various impurity components which might influence quantitative accuracy, and examples of the impurity components include polychlorinated polycyclic aromatic hydrocarbons having chemical structures or chemical behaviors similar to those of the dioxins, such as polychlorinated diphenyl ether (PCDE) and PCBs (hereinafter sometimes referred to as "non-DL-PCBs") other than the DL-PCBs. For this reason, the extracts with the dioxins are normally purified and concentrated as necessary after purification, and finally, are applied to an analytical instrument. As an extract purification method, Patent Literature 1 describes a method using a column chromatography including a first-stage column filled with sulfuric silica gel and silver nitrate silica gel as a purifier and a second-stage column filled with activated carbon-containing silica gel or graphite carbon as an adsorbent. In this method, the activated carbon silica gel or the graphite carbon can be selectively used as the adsorbent of the second-stage column. In the case of using both of the activated carbon silica gel or the graphite carbon, each filler can be used in a stacked state or a mixed state.

In the purification method using the column chromatography, the extracts containing the dioxins are first applied into the first-stage column, and a hydrocarbon solvent is subsequently supplied to the first-stage column. The hydrocarbon solvent dissolves the dioxins in the extracts while passing through the first-stage column and the second-stage column. At this point, the dioxins dissolved in the hydrocarbon solvent pass through the purifier of the first-stage column, and adsorb to the adsorbent of the second-stage column. Generally, the impurity components contained in the extracts are dissolved in the hydrocarbon solvent together with the dioxins. Some of the impurity components are decomposed and other impurity components adsorb to the purifier while passing through the purifier of the first-stage column. Of the impurity components or decomposition products thereof, those not adsorbing to the purifier pass through the adsorbent of the second-stage column in a state in which these components are dissolved in the hydrocarbon solvent, and are discharged from the column.

Next, the first-stage column and the second-stage column are separated from each other, and alkyl benzene capable of dissolving the dioxins is supplied to the second-stage column. Then, the dioxins adsorbed on the second-stage column, from which the impurity components have been separated, are desorbed by eluting the dioxins by an alkyl benzene solution. Such dioxins eluted by an alkyl benzene solution, after having been concentrated as necessary, are analyzed by the GC/MS.

In such a purification method, all types of dioxins contained in the extracts are trapped by the adsorbent of the second-stage column, and these dioxins are eluted by using the alkyl benzene. Thus, in the GC/MS measurement, all types of dioxins contained in the alkyl benzene solution can be simultaneously analyzed.

However, when the alkyl benzene solution containing all types of dioxins is analyzed simultaneously by GC/MS, quantitative accuracy of an analysis might lack reliability due to mutual interference between several PCDDs isomers and some of PCBs. For example, in the case of analyzing all type of dioxins by a high-resolution GC/MS, it has been known that the mono-ortho PCBs influence a result of quantitative analysis of the PCDDs and the PCDFs, and conversely, the PCDDs and the PCDFs influence a result of quantitative analysis of the mono-ortho PCBs.

For this reason, in general, the dioxins have been fractionated into several compound groups by some adsorbents. For example, Patent Literature 2 describes a method in which graphite-like carbon or a mixture of graphite-like carbon and other materials such as silica gel, activated carbon-containing silica gel, activated carbon, alumina, and zeolite is used as an adsorbent for the dioxins.

In this method, a purified dioxin solution is supplied to a column filled with the adsorbent such that the dioxins adsorb to the adsorbent. Then, several types of solvents are sequentially supplied to the column, thereby preparing several types of dioxin solutions. Patent Literature 2 describes that by such a method, three types of dioxin solutions including a solution containing the PCBs other than the DL-PCBs, a solution containing the mono-ortho PCBs, and a solution containing the non-ortho PCBs, the PCDDs, and the PCDFs can be prepared, for example.

However, in this method, all types of dioxins adsorb to the adsorbent as in the method described in Patent Literature 1, and therefore, it is difficult to precisely fractionate the dioxins. For example, there is a probability that the fraction containing non-ortho PCBs, PCDDs and PCDFs is contaminated with some of the mono-ortho PCBs, and there is also the opposite situation as well.

### PRIOR ART LITERATURE

### PATENT LITERATURE

Patent Literature 1: JP-A-2002-40007
Patent Literature 2: JP-A-2006-297368

### DISCLOSURE OF THE INVENTION

### PROBLEMS TO BE SOLVED BY THE INVENTION

The present invention is intended to fractionate, with a high accuracy, dioxins into a dioxin group including non-ortho PCBs, PCDDs, and PCDFs and mono-ortho PCBs.

### SOLUTIONS TO THE PROBLEMS

The present invention relates to the method for fractionating dioxins, and the fractionating method includes the step of causing an aliphatic hydrocarbon solvent solution with the dioxins to pass through an adsorbent layer using an adsorbent mixed active magnesium silicate.

In such a fractionating method, when the aliphatic hydrocarbon solvent solution with the dioxins passes through the adsorbent layer, a dioxin group including non-ortho PCBs, PCDDs, and PCDFs adsorbs, among the dioxins, to the adsorbent containing the active magnesium silicate. On the other hand, mono-ortho PCBs of the dioxins remain in the aliphatic hydrocarbon solvent solution, and passes through the adsorbent layer. As a result, the dioxins in the aliphatic hydrocarbon solvent solution are fractionated into the dioxin group, which is trapped by the adsorbent layer, including the non-ortho PCBs, the PCDDs, and the PCDFs and the mono-ortho PCBs remaining in the aliphatic hydrocarbon solvent solution.

One aspect of the adsorbent used in the fractionating method further contains graphite mixed with the active magnesium silicate. The content of the graphite in the adsorbent of this aspect is preferably equal to or lower than 25% by weight. Moreover, the active magnesium silicate in the adsorbent of this aspect is, for example, prepared in such a manner that a mixture of magnesium silicate and graphite is heated to equal to or lower than 650°C.

In one aspect of the fractionating method of the present invention, the aliphatic hydrocarbon solvent solution having passed through the adsorbent layer further passes through an alumina layer.

In the fractionating method of this aspect, when the aliphatic hydrocarbon solvent solution having passed through the adsorbent layer passes through the alumina layer, the remaining mono-ortho PCBs adsorb to the alumina layer. As a result, the dioxins in the aliphatic hydrocarbon solvent solution are fractionated into the dioxin group, which is selectively trapped in the adsorbent layer, including the non-ortho PCBs, the PCDDs, and the PCDFs and the mono-ortho PCBs selectively trapped in the alumina layer.

The fractionating method of this aspect may further include the step of supplying a solvent capable of dissolving the dioxins to the adsorbent layer through which the aliphatic hydrocarbon solvent solution has passed to collect the solvent having passed through the adsorbent layer and the step of supplying a solvent capable of dissolving the dioxins to the alumina layer through which the aliphatic hydrocarbon solvent solution has passed to collect the solvent having passed through the alumina layer.

In a case where the fractionating method of this aspect further includes these steps, the dioxin group, which is trapped by the adsorbent layer, including the non-ortho PCBs, the PCDDs, and the PCDFs and the mono-ortho PCBs trapped by the alumina layer are dissolved in the solvents supplied to each layer to dissolve the dioxins, and are extracted from each layer. Then, these dioxins are obtained as separate extracts.

The present invention according to another aspect relates to a tool for fractionating dioxins contained in a dioxin solution. The fractionating tool includes a pipe body opening at both ends and an adsorbent layer filled into the pipe body and using an adsorbent mixed active magnesium silicate.

When the dioxins contained in the dioxin solution are fractionated using such a fractionating tool, the dioxin solution is added to the adsorbent layer of the pipe body. Then, when an aliphatic hydrocarbon solvent capable of dissolving the dioxins is supplied to the adsorbent layer to which the dioxin solution has been added, the aliphatic hydrocarbon solvent dissolves the dioxins and turns into an aliphatic hydrocarbon solvent with the dioxins, and then, passes through the adsorbent layer. At this point, the dioxins are dissolved in the adsorbent layer, and among these dioxins, a dioxin group including non-ortho PCBs, PCDDs, and PCDFs adsorbs to the adsorbent. The aliphatic hydrocarbon solvent passes through the adsorbent layer in a state in which mono-ortho PCBs are dissolved in the aliphatic hydrocarbon solvent. As a result, the dioxins contained in the dioxin solution are fractionated into the dioxin group including the non-ortho PCBs, the PCDDs, and the PCDFs and the mono-ortho PCBs.

In one aspect of the fractionating tool, the adsorbent further contains graphite mixed with the active magnesium silicate. In this case, the content of the graphite in the adsorbent is preferably equal to or lower than 25% by weight.

One aspect of the fractionating tool of the present invention further includes an alumina layer filled into the pipe body. One example of the pipe body in the fractionating tool of this aspect has an opening between the adsorbent layer and the alumina layer.

In the fractionating tool of this aspect, the mono-ortho PCBs contained in the aliphatic hydrocarbon solvent having passed through the adsorbent layer adsorb to the alumina layer, and are separated from the aliphatic hydrocarbon solvent. As a result, the dioxins in the dioxin solution are fractionated into the dioxin group, which is selectively trapped by the adsorbent layer, including the non-ortho PCBs, the PCDDs, and the PCDFs and the mono-ortho PCBs selectively trapped by the alumina layer.

The present invention according to still another aspect relates to the method for preparing a sample for analyzing dioxins contained in a dioxin solution. Such a preparation method includes the step of adding the dioxin solution to a purification layer including a silver nitrate silica gel layer and a sulfuric silica gel layer, the step of supplying an aliphatic hydrocarbon solvent to the purification layer to which the dioxin solution has been added, the step of causing the aliphatic hydrocarbon solvent having passed through the purification layer to pass through an adsorbent layer using an adsorbent mixed active magnesium silicate, the step of causing the aliphatic hydrocarbon solvent having passed through the adsorbent layer to pass through an alumina layer, the step of supplying a solvent capable of dissolving the dioxins to the alumina layer through which the aliphatic hydrocarbon solvent has passed to collect, as a first analysis sample, the solvent having passed through the alumina layer, and the step of supplying a solvent capable of dissolving the dioxins to the adsorbent layer through which the aliphatic hydrocarbon solvent has passed to collect, as a second analysis sample, the solvent having passed through the adsorbent layer.

In such a preparation method, when the aliphatic hydrocarbon solvent is supplied to the purification layer to which the dioxin solution has been added, the aliphatic hydrocarbon solvent passes through the purification layer. At this point, the dioxins and impurity components contained in the dioxin solution are dissolved in the aliphatic hydrocarbon solvent. Then, some of the impurity components react with the silver nitrate silica gel layer or the sulfuric silica gel layer of the purification layer, and are decomposed. Moreover, some of the impurity components and decomposition products adsorb to the silver nitrate silica gel layer or the sulfuric silica gel layer. Meanwhile, the dioxins pass through the purification layer in a state in which the dioxins are dissolved in the aliphatic hydrocarbon solvent. As a result, the dioxins are separated from some of the impurity components.

When the aliphatic hydrocarbon solvent having passed through the purification layer and having dissolved the dioxins passes through the adsorbent layer, a dioxin group including non-ortho PCBs, PCDDs, and PCDFs selectively adsorb, among the dioxins, to the adsorbent. When passing through the alumina layer, mono-ortho PCBs of the dioxins selectively adsorb to the alumina layer. Thus, the first analysis sample is an analysis sample for the mono-ortho PCBs, and the second analysis sample is an analysis sample for the dioxin group including the non-ortho PCBs, the PCDDs, and the PCDFs. That is, according to the preparation method, the analysis sample for the mono-ortho PCBs and the analysis sample for the dioxin group including the non-ortho PCBs, the PCDDs, and the PCDFs can be separately prepared.

The present invention according to still another aspect relates to the method for determining dioxins contained in a dioxin solution. Such a determination method includes the step of separately analyzing, by a gas chromatography method or a bioassay method, the first analysis sample and the second analysis sample prepared by the method for preparing the analysis sample for the dioxins according to the present invention.

In such a determination method, mono-ortho PCBs can be analyzed by analysis of the first analysis sample, and non-ortho PCBs, PCDDs, and PCDFs can be analyzed by analysis of the second analysis sample.

### EFFECTS OF THE INVENTION

The method for fractionating the dioxins according to the present invention uses the adsorbent layer using the adsorbent containing the active magnesium silicate. Thus, the dioxins can be fractionated into the dioxin group including the non-ortho PCBs, the PCDDs, and the PCDFs and the mono-ortho PCBs, and the accuracy of such fractionation can be enhanced.

The dioxin fractionating tool according to the present invention includes the adsorbent layer using the adsorbent containing the active magnesium silicate. Thus, the dioxins can be fractionated into the dioxin group including the non-ortho PCBs, the PCDDs, and the PCDFs and the mono-ortho PCBs, and the accuracy of such fractionation can be enhanced.

The method for preparing the analysis sample for the dioxins according to the present invention uses the adsorbent layer using the adsorbent containing the active magnesium silicate. Thus, the analysis sample for the mono-ortho PCBs and the analysis sample for the dioxin group including the non-ortho PCBs, the PCDDs, and the PCDFs can be separately prepared from the dioxin solution.

The method for determining the dioxins according to the present invention includes the step of separately analyzing the first analysis sample and the second analysis sample prepared by the method for preparing the analysis sample for the dioxins according to the present invention. Thus, the mono-ortho PCBs can be analyzed by analysis of the first analysis sample, and the non-ortho PCBs, the PCDDs, and the PCDFs can be analyzed by analysis of the second analysis sample.

### BRIEF DESCRIPTION OF THE DRAWINGS

Fig. 1 is a partial sectional view of the outline of a first embodiment of an apparatus for performing an analysis sample preparation method according to the present invention.
Fig. 2 is a partial sectional view of the outline of a variation of the apparatus illustrated in Fig. 1.
Fig. 3 is a partial sectional view of the outline of a second embodiment of the apparatus for performing the analysis sample preparation method according to the present invention.
Fig. 4 is a sectional view illustrating the outline of one example of a fractionating tool for performing the analysis sample preparation method according to the present invention.
Fig. 5 is a sectional view illustrating the outline of part of a variation of the fractionating tool illustrated in Fig. 4.

### DESCRIPTION OF PREFERRED EMBODIMENTS

Embodiments of an analysis sample preparation method according to the present invention will be described below with reference to the figures. Each figure illustrates the outline of an example of an apparatus used for performing the preparation method of the present invention or a fractionating tool, and the structure, shape, size and the like of each unit are not precisely reflected on each figure.

### First Embodiment

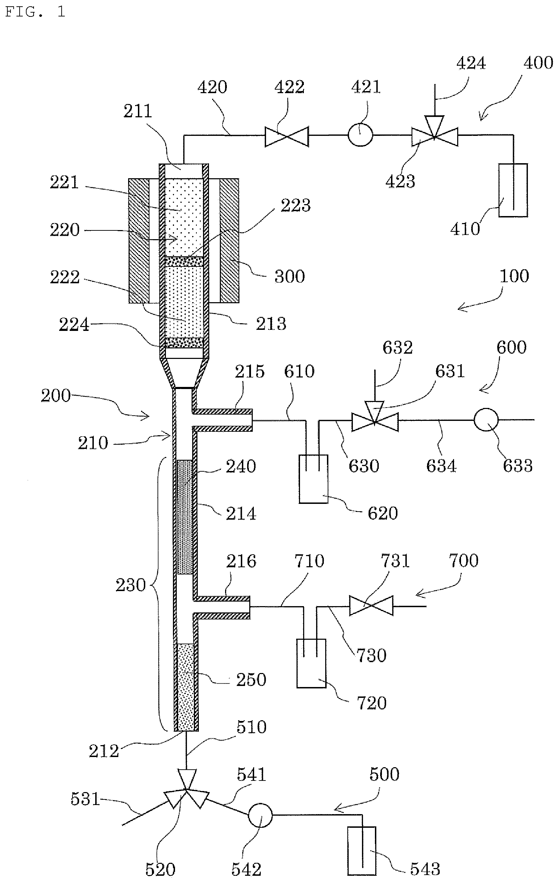

A first embodiment of an apparatus capable of performing the analysis sample preparation method according to the present invention will be described with reference to Fig. 1. In Fig. 1, a preparation apparatus 100 is for preparing a dioxin analysis sample from a dioxin solution, and mainly includes a dioxin fractionating tool 200, a heating apparatus 300, a solvent supply apparatus 400, a solvent outflow path 500, a first extraction path 600, and a second extraction path 700.

The fractionating tool 200 includes a pipe body 210. The pipe body 210 is made of a material having at least solvent resistance, chemical resistance, and thermal resistance, such as glass, resin, or metal having these properties. The pipe body 210 is formed in a continuous cylindrical shape opening at both ends, the pipe body 210 having an opening 211 at one end and having an opening 212 at the other end. The pipe body 210 has a large-diameter portion 213 formed on an opening 211 side and set to have a relatively-large diameter and a small-diameter portion 214 formed on an opening 212 side and set to have a relatively-small diameter. The small-diameter portion 214 has two branched paths as openings, i.e., a first branched path 215 and a second branched path 216 provided with a clearance.

The pipe body 210 is held in a standing state, and is filled with a purification layer 220 and a fractionating layer 230.

The large-diameter portion 213 is filled with the purification layer 220, and the purification layer 220 is a multilayer silica gel layer in which a silver nitrate silica gel layer 221, a first active silica gel layer 223, a sulfuric silica gel layer 222, and a second active silica gel layer 224 are arranged in this order from the opening 211 side.

The silver nitrate silica gel layer 221 is made of silver nitrate silica gel, and is provided for decomposing or adsorbing some of impurity components mixed with the dioxin solution. The silver nitrate silica gel used herein is prepared in such a manner that after a silver nitrate solution has been uniformly added to a surface of silica gel (normally active silica gel of which degree of activity has been enhanced by heating) in the form of grain with a particle size of about 40 to 210 µm, moisture is removed by heating under a reduced pressure. The amount of the silver nitrate solution added to the silica gel is normally preferably set to 5 to 20% of the weight of the silica gel.

The density of the filled silver nitrate silica gel in the silver nitrate silica gel layer 221 is not specifically limited, but is normally preferably set to 0.3 to 0.8 g/cm3 and more preferably 0.4 to 0.7 g/cm3.

The sulfuric silica gel layer 222 is made of sulfuric silica gel, and is provided for decomposing or adsorbing some of the impurity components mixed with the dioxin solution other than dioxins. The sulfuric silica gel used herein is prepared in such a manner that concentrated sulfuric acid is uniformly added to a surface of silica gel (normally active silica gel of which degree of activity has been enhanced by heating) in the form of grain with a particle size of about 40 to 210 µm. The amount of the concentrated sulfuric acid added to the silica gel is normally preferably set to 10 to 130% of the weight of the silica gel.

The density of the filled sulfuric silica gel in the sulfuric silica gel layer 222 is not specifically limited, but is normally preferably set to 0.3 to 1.1 g/cm3 and more preferably 0.5 to 1.0 g/cm3.

The first active silica gel layer 223 is arranged to avoid chemical reaction between the silver nitrate silica gel layer 221 and the sulfuric silica gel layer 222 due to direct contact therebetween, and is made of silica gel in the form of grain with a particle size of about 40 to 210 µm. The silica gel used herein may be one of which degree of activity has been enhanced as necessary by heating.

The second active silica gel layer 224 is made of silica gel similar to that of the first active silica gel layer 223, and is provided for adsorbing some of the impurity components decomposed due to reaction with the sulfuric silica gel layer 222, a decomposition product, and sulfuric silica eluted from the sulfuric silica gel layer 222 to prevent these components from moving to the fractionating layer 230.

In the purification layer 220, a ratio between the silver nitrate silica gel layer 221 and the sulfuric silica gel layer 222 is set such that the weight ratio of the sulfuric silica gel layer 222 to the silver nitrate silica gel layer 221 is preferably set to 1.0 to 50 and more preferably 3.0 to 30. When the weight ratio of the sulfuric silica gel layer 222 exceeds 50, the percentage of the silver nitrate silica gel layer 221 is relatively low, and for this reason, there is a probability that the capacity of the purification layer 220 for adsorbing the impurity components contained in the dioxin solution is insufficient. Conversely, the weight ratio of the sulfuric silica gel layer 222 is lower than 1.0, there is a probability that the capacity of the purification layer 220 for decomposing the impurity components contained in the dioxin solution is insufficient.

The fractionating layer 230 is provided for fractionating the dioxins contained in the dioxin solution, and includes an adsorbent layer 240 using an adsorbent mixed active magnesium silicate and an alumina layer 250. The small-diameter portion 214 is filled with the adsorbent layer 240 and the alumina layer 250 such that the adsorbent layer 240 and the alumina layer 250 are provided with a clearance. More specifically, the small-diameter portion 214 is, between the first branched path 215 and the second branched path 216, filled with the adsorbent layer 240. The small-diameter portion 214 is, between the second branched path 216 and the opening 212, filled with the alumina layer 250.

The active magnesium silicate contained in the adsorbent used in the adsorbent layer 240 is for removing moisture by heating of magnesium silicate and enhancing an adsorption capacity accordingly. The magnesium silicate described herein is silicate salt that an electronegative atomic group containing oxygen and magnesium around a silicon atom is coordinated, and generally represented by a chemical formula xMgO·ySiO2. The magnesium silicate includes various compositions with different combinations of x and y, and may be hydrate (in this case, represented by a chemical formula xMgO·ySiO2·nH2O). As representative examples of the combination (x:y) of x and y in sodium silicate, 2:5, 2:3, and 3:4 have been known. Of these combinations, one that x:y is 2:3 is preferably used.

As the active magnesium silicate, one obtained in such a manner that porous magnesium silicate capable of ensuring liquid permeability in the adsorbent layer 240 and provided in the form of grain or powder, such as magnesium silicate with a particle size of 38 to 250 µm (60 to 390 mesh) or specifically magnesium silicate with a particle size of 75 to 150 µm (100 to 200 mesh), is heated and activated is preferred. The magnesium silicate in the form of grain or powder is, for example, commercially available by multiple companies under the name of "Florisil," and these commercially-available products can be used.

Heating treatment for activating the magnesium silicate is, for example, preferably executed at 650°C or lower under the flow of inert gas such as nitride by means of a tubular furnace, and is specifically preferably executed at 500°C or lower. At this point, the flow rate of the inert gas is preferably set to 0.5 to 1.0 L/minute. Moreover, heating time is preferably set to 0.5 to 3 hours, and is specifically preferably set to 1 to 2 hours. The magnesium silicate heated under a temperature condition exceeding 650°C is altered beyond the range of activation by moisture removal, and for this reason, fractionation into a dioxin group including non-ortho PCBs, PCDDs, and PCDFs and mono-ortho PCBs becomes difficult.

The adsorbent used in the adsorbent layer 240 may further contain graphite mixed with the active magnesium silicate. In the case of using such an adsorbent, the dioxin analysis sample fractionated into an analysis sample for the dioxin group including the non-ortho PCBs, the PCDDs, and the PCDFs and an analysis sample for the mono-ortho PCBs with a higher accuracy can be prepared from the dioxin solution.

As the graphite, various commercially-available products can be used. Normally, one provided in the form of grain or powder with a particle size of about 40 to 200 µm and formed such that the specific surface area thereof measured by a BET method is 10 to 500 m2/g, specifically 50 to 200 m2/g, is preferred. Moreover, as the graphite, one heated or rinsed with an organic solvent for removing an organic compound remaining as an impure component is preferred.

The percentage of the graphite in the adsorbent is preferably equal to or lower than 25% by weight, more preferably equal to or lower than 20% by weight, much more preferably equal to or lower than 15% by weight, and specifically preferably equal to or lower than 12.5% by weight. In a case where the percentage of the graphite exceeds 25% by weight, there is a probability that the capacity of the adsorbent layer 240 for adsorbing the mono-ortho PCBs is enhanced and the capacity of the adsorbent layer 240 for fractionation into the dioxin group including the non-ortho PCBs, the PCDDs, and the PCDFs and the mono-ortho PCBs is degraded.

The mixture of the active magnesium silicate and the graphite in the adsorbent may be one obtained in such a manner that a mixture of the magnesium silicate and the graphite is heated to activate the magnesium silicate in such a mixture. In a case where the dioxin solution contains an impurity component derived from an environmental component, specifically a case where an aromatic hydrocarbon compound derived from the environmental component is contained as the impurity component, tendency shows that the capacity for fractionation into the dioxin group including the non-ortho PCBs, the PCDDs, and the PCDFs and the mono-ortho PCBs is degraded due to influence of the impurity component. However, the above-described mixture is used in the absorbent so that the fractionation capacity can be enhanced.

The treatment for heating the mixture of the magnesium silicate and the graphite is, for example, preferably executed at 650°C or lower under the flow of inert gas such as nitrogen by means of a tubular furnace, and is specifically preferably executed at 500°C or lower. At this point, the flow rate of the inert gas is preferably set to 0.5 to 1.0 L/minute. Moreover, heating time is preferably set to 0.5 to 3 hours, and is specifically preferably set to 1 to 2 hours. In a case where a temperature condition for the heating treatment exceeds 650°C, the magnesium silicate is altered beyond the range of activation by moisture removal, and fractionation into the dioxin group including the non-ortho PCBs, the PCDDs, and the PCDFs and the mono-ortho PCBs becomes difficult.

The density of the filled adsorbent in the adsorbent layer 240 is not specifically limited, but is normally preferably set to 0.2 to 0.6 g/cm3 and more preferably 0.3 to 0.5 g/cm3.

The alumina layer 250 is filled with alumina in the form of grain. The alumina used herein may be any of basic alumina, neutral alumina, and acidic alumina. The degree of activity of the alumina is not specifically limited. The preferred particle size of the alumina is normally 40 to 300 µm.

The density of the filled alumina in the alumina layer 250 is not specifically limited, but is normally preferably set to 0.5 to 1.2 g/cm3 and more preferably 0.8 to 1.1 g/cm3.

An amount ratio (A:B) between the adsorbent layer 240 (A) and the alumina layer 250 (B) is normally preferably set to 1:0.5 to 1:3 in terms of volume ratio and more preferably 1:1 to 1:2, considering enhancement of fractionation accuracy and reduction in the probability of some of the dioxins leaking without being trapped by the fractionating layer 230.

The size of the fractionating tool 200 can be set as necessary according to the amount of dioxin solution treated by the preparation apparatus 100, and is not specifically limited. For example, in a case where the dioxin solution amount is about 1 to 20 mL, the large-diameter portion 213 is preferably configured such that a portion fillable with the purification layer 220 has an inner diameter of 10 to 20 mm and a length of about 100 to 300 mm. Moreover, the small-diameter portion 214 preferably has an inner diameter of 3 to 10 mm, and is preferably configured such that the length of a portion fillable with the adsorbent layer 240 is about 20 to 80 mm and the length of a portion fillable with the alumina layer 250 is about 20 to 80 mm.

The heating apparatus 300 is arranged to surround the outer periphery of the large-diameter portion 213, and is provided for heating the silver nitrate silica gel layer 221 and the first active silica gel layer 223 of the purification layer 220 and part of the sulfuric silica gel layer 222, i.e., a portion in the vicinity of the silver nitrate silica gel layer 221.

The solvent supply apparatus 400 has a first solvent supply path 420 extending from a first solvent container 410 to the pipe body 210. The first solvent supply path 420 is detachable from the opening 211 of the pipe body 210, and when attached to the opening 211, can air-tightly close the opening 211. The first solvent supply path 420 has, in this order from a first solvent container 410 side, an air introduction valve 423 and a first pump 421 and a first valve 422 for supplying a solvent stored in the first solvent container 410 to the pipe body 210. The air introduction valve 423 is a three-way valve having an air introduction path 424 opening at one end, and is provided for switching a flow path to an air introduction path 424 side or the first solvent container 410 side. The first valve 422 is a two-way valve, and is provided for switching the first solvent supply path 420 between an open state and a closed state.

The solvent stored in the first solvent container 410 is an aliphatic hydrocarbon solvent capable of dissolving the dioxins and preferably a saturated aliphatic hydrocarbon solvent with a carbon number of 5 to 8. Examples of the solvent include n-pentane, n-hexane, n-heptane, n-octane, iso-octane, and cyclohexane. These solvents may be used as a mixture, as necessary.

The solvent outflow path 500 has a flow path 510 air-tightly connected to the opening 212 of the pipe body 210. The flow path 510 has a second valve 520. The second valve 520 is a three-way valve, and a discarding path 531 for discarding a solvent from the pipe body 210 and a second solvent supply path 541 for supplying a solvent to the pipe body 210 communicate with the flow path 510. The flow path 510 is provided for switching such communication to communication to either one of the discarding path 531 or the second solvent supply path 541.

The second solvent supply path 541 has a second pump 542, and communicates with a second solvent container 543 configured to store a solvent for extracting the dioxins trapped by the fractionating tool 200. The extraction solvent stored in the second solvent container 543 can be selected according to a later-described dioxin determination method. In a case where a gas chromatography method is employed as the determination method, a solvent suitable for such a method, such as toluene or benzene, can be used. Alternatively, a solvent mixture obtained in such a manner that an aliphatic hydrocarbon solvent or an organic chlorine-based solvent is added to the toluene or the benzene can be used. In the case of using the solvent mixture, the percentage of the toluene or the benzene is set to equal to or higher than 50% by weight. Examples of the aliphatic hydrocarbon solvent used in the solvent mixture include n-pentane, n-hexane, n-heptane, n-octane, iso-octane, and cyclohexane. Examples of the organic chlorine-based solvent include dichloromethane, trichloromethane, and tetrachloromethane. Of these extraction solvents, the toluene is specifically preferred because the dioxins can be extracted from the fractionating tool 200 by use of a small amount of the solvent.

In a case where a bioassay method is employed as the determination method, a solvent suitable for such a method, such as hydrophilic solvents including dimethylsulfoxide (DMSO) and methanol, is used.

The first extraction path 600 has a first recovery path 610 extending from the first branched path 215. The first recovery path 610 air-tightly communicates the first branched path 215 at one end, and at the other end, is air-tightly inserted into a first recovery container 620 for recovering a solvent. One end of a first ventilation path 630 is, independently of the first recovery path 610, air-tightly inserted into the first recovery container 620. The first ventilation path 630 includes, at the other end, a third valve 631. The third valve 631 is a three-way valve, and communicates with an open path 632 opening at one end and an air supply path 634 including a compressor 633 for sending compressed air to the first ventilation path 630. The third valve 631 is provided for switching the first ventilation path 630 to communicate with either one of the open path 632 or the air supply path 634.

The second extraction path 700 has a second recovery path 710 extending from the second branched path 216. The second recovery path 710 air-tightly communicates with the second branched path 216 at one end, and at the other end, is air-tightly inserted into a second recovery container 720 for recovering a solvent. One end of a second ventilation path 730 is, independently of the second recovery path 710, air-tightly inserted into the second recovery container 720. The second ventilation path 730 includes a fourth valve 731. The fourth valve 731 is a two-way valve, and is provided for switching the second ventilation path 730 between an open state and a closed state.

Next, the method for preparing the dioxin analysis sample from the dioxin solution by means of the above-described preparation apparatus 100 will be described. First, in the preparation apparatus 100, the first valve 422, the air introduction valve 423, the second valve 520, the third valve 631, and the fourth valve 731 are set to prescribed initial states. That is, the first valve 422 is set to an open state, and the air introduction valve 423 is set to communicate with the first solvent container 410 side. Moreover, the second valve 520 is set such that the flow path 510 communicates with the discarding path 531. Further, the third valve 631 is set such that the first ventilation path 630 and the air supply path 634 communicate with each other, and the fourth valve 731 is set to a closed state.

The analysis sample preparation method mainly includes dioxin fractionating and extraction steps.

### <Dioxin Fractionating Step>

After setting to the initial states, the dioxin solution is applied into the fractionating tool 200. At this point, the first solvent supply path 420 is detached from the pipe body 210, and the dioxin solution is applied into the purification layer 220 through the opening 211. Then, after the pipe body 210 has been attached to the first solvent supply path 420, the heating apparatus 300 is actuated to heat part of the purification layer 220, i.e., the entirety of the silver nitrate silica gel layer 221 and the first active silica gel layer 223 and part of the sulfuric silica gel layer 222.

The dioxin solution applied herein is, for example, an extract obtained in such a manner that dioxins are, using a solvent, extracted from a sample which might contain the dioxins, such as an environmental sample including ambient air and soil or a food sample. However, the dioxin solution may be oil-like food which might contain dioxins, such as fish oil.

Such a dioxin solution often contains, as the impurity components, polychlorinated polycyclic aromatic hydrocarbons such as PCDE and non-DL-PCBs and other aromatic hydrocarbon compounds which have chemical structures or chemical behaviors similar to those of the dioxins and have the possibility of influencing a dioxin quantitative accuracy. Specifically, an extract with dioxins from an environmental sample such as soil, exhaust gas from a combustion furnace or the like, a bottom material, or sludge often contains, as the impurity components, various organic compounds which are less likely to be separated from the dioxins, and typically contains an aromatic hydrocarbon compound. These impurity compounds are highly likely to influence the accuracy of fractionating the dioxins into the dioxin group including the non-ortho PCBs, the PCDDs, and the PCDFs and the mono-ortho PCBs. In the case of the extracts with dioxins from the soil, such extracts often contain, as the impurity components, paraffins (straight-chain hydrocarbon compounds) contained much in the soil. The paraffins are likely to adsorb to a carbon-based adsorbent together with the PCDDs, the PCDFs, and the non-ortho PCBs, and are likely to be extracted from the adsorbent together with the PCDDs, the PCDFs, and the non-ortho PCBs. For this reason, the paraffins are known as a substance responsible for lock mass fluctuation influencing the analysis accuracy in the case of analyzing the dioxins by a GC/MS method (specifically a GC-HRMS method).

The extracts with dioxins can be directly applied into the fractionating tool 200 as long as the aliphatic hydrocarbon solvent is used for such a solution. In a case where the extract is obtained by extraction using an organic solvent other than the aliphatic hydrocarbon solvent, such as the aromatic hydrocarbon solvent including the toluene, such an extract can be applied into the fractionating tool 200 after the aromatic hydrocarbon solvent used for extraction has been substituted for the aliphatic hydrocarbon solvent. The aliphatic hydrocarbon solvent used for extraction or solvent substitution is normally preferably an aliphatic hydrocarbon solvent with a carbon number of 5 to 10. Examples of the aliphatic hydrocarbon solvent include n-hexane, iso-octane, nonane, and decane. Specifically, inexpensive n-hexane is preferred.

The amount of dioxin solution applied into the fractionating tool 200 is normally preferably about 1 to 10 mL. The solution to be applied can be concentrated in such a manner that part of the solvent is distilled.

In a case where the dioxin solution is in the form of oil such as fish oil, such a dioxin solution can be applied into the fractionating tool 200 together with the aliphatic hydrocarbon solvent which can dissolve the dioxin solution, or can be applied into the fractionating tool 200 as a solution in which the solvent has been dissolved in advance. In this case, the total amount of dioxin solution and aliphatic hydrocarbon solvent is set to the above-described injection amount.

The applied dioxin solution penetrates an upper portion of the silver nitrate silica gel layer 221, and is heated by the heating apparatus 300 together with part of the purification layer 220. The temperature of heating by the heating apparatus 300 is equal to or higher than 35°C, preferably equal to or higher than 50°C, and more preferably equal to or higher than 60°C. By such heating, some of the impurity components in the solution other than the dioxins react with the purification layer 220, and are decomposed. In a case where the heating temperature is lower than 35°C, reaction among the impurity components and the purification layer 220 is less likely to progress, and there is a probability that some of the impurity components easily remain in the dioxin analysis sample. The upper limit of the heating temperature is not specifically limited, but is normally preferably equal to or lower than a boiling temperature in terms of safety.

Upon heating, the silver nitrate silica gel layer 221 and the sulfuric silica gel layer 222 are stacked with the first active silica gel layer 223 being interposed therebetween, and therefore, reaction therebetween is reduced.

Next, the solvent is supplied from the solvent supply apparatus 400 to the fractionating tool 200 after a lapse of 10 to 60 minutes from the start of heating. At this point, the heating apparatus 300 may be kept actuated, or may be stopped. At this step, the first pump 421 is actuated with the first valve 422 being maintained in the open state, thereby supplying a moderate amount of solvent stored in the first solvent container 410 into the pipe body 210 through the opening 211 by way of the first solvent supply path 420. This solvent dissolves the dioxins, impurity component decomposition products, and remaining undecomposed impurity components (these impurity components normally include the non-DL-PCBs) in the dioxin solution, and as the aliphatic hydrocarbon solvent solution containing the dioxins, passes through the purification layer 220. At this point, the decomposition products and some of the impurity components adsorb to the silver nitrate silica gel layer 221, the first active silica gel layer 223, the sulfuric silica gel layer 222, and the second active silica gel layer 224. Moreover, the solvent passing through the purification layer 220 is naturally cooled by an unheated portion of the heating apparatus 300, i.e., a lower portion of the sulfuric silica gel layer 222 and the second active silica gel layer 224.

The solvent having passed through the purification layer 220 flows toward the fractionating layer 230, and passes through the adsorbent layer 240 and the alumina layer 250. The solvent flows into the flow path 510 through the opening 212, and is discarded through the discarding path 531. At this point, the dioxins contained in the solvent from the purification layer 220 are trapped by the fractionating layer 230, and are separated from the solvent. More specifically, in the fractionating layer 230, the non-ortho PCBs, the PCDDs, and the PCDFs of the dioxins adsorb to the adsorbent layer 240, and the mono-ortho PCBs adsorb to the alumina layer 250. Thus, the dioxins contained in the solvent is, in the fractionating layer 230, fractionated into the dioxin group including the non-ortho PCBs, the PCDDs, and the PCDFs and the mono-ortho PCBs.

Some of the impurity components contained in the solvent having passed through the purification layer 220 pass through the fractionating layer 230 and are discarded together with the solvent. Some other impurity components are trapped by the fractionating layer 230. For example, the non-DL-PCBs and the PCDE adsorb, together with the mono-ortho PCBs, to the alumina layer 250. Moreover, the aromatic organic compounds and the paraffins pass through the fractionating layer 230, and are discarded through the discarding path 531.

### <Dioxin Extraction Step>

Next, the dioxins having adsorbed to the fractionating layer 230 are extracted using the solvent, and the dioxin analysis sample is prepared. Before such preparation, the purification layer 220 and the fractionating layer 230 are dried in the preparation apparatus 100. At this point, the air introduction valve 423 of the solvent supply apparatus 400 is first switched to the air introduction path 424 side. Then, the first pump 421 is actuated to suck air from the air introduction path 424.

The air sucked from the air introduction path 424 is supplied into the pipe body 210 through the opening 211 by way of the first solvent supply path 420. The air passes through the purification layer 220 and the fractionating layer 230 to flow into the flow path 510 through the opening 212, and is discharged through the discarding path 531. At this point, the solvent remaining in the purification layer 220 is pushed out by the passing air, and moves to the fractionating layer 230. As a result, the purification layer 220 is dried.

Next, the first pump 421 is stopped, and the first valve 422 is switched to a closed state. Then, the compressor 633 is actuated on the first extraction path 600.

By actuation of the compressor 633, compressed air is supplied into the first branched path 215 from the air supply path 634 through the first ventilation path 630, the first recovery container 620, and the first recovery path 610. Such compressed air passes through the fractionating layer 230, and flows into the flow path 510 through the opening 212. The compressed air is discharged through the discarding path 531. At this point, the solvent having moved from the purification layer 220 to the fractionating layer 230 and the solvent remaining in each layer of the fractionating layer 230 are pushed out by the compressed air, and are discharged through the discarding path 531 together with the compressed air. As a result, each layer of the fractionating layer 230 is dried.

At a first step for preparing the dioxin analysis sample, the compressor 633 is stopped, and the fourth valve 731 of the second extraction path 700 is switched to an open state. On the solvent outflow path 500, the second valve 520 is switched such that the flow path 510 communicates with the second solvent supply path 541, and the second pump 542 is actuated. Accordingly, a moderate amount of solvent stored in the second solvent container 543 is supplied into the pipe body 210 through the second solvent supply path 541 and the flow path 510 through the opening 212.

The solvent supplied into the pipe body 210 through the flow path 510 flows into the second branched path 216 through the alumina layer 250, and is recovered by the second recovery container 720 through the second recovery path 710 of the second extraction path 700. At this point, the solvent dissolves the mono-ortho PCBs and the non-DL-PCBs having adsorbed to the alumina layer 250, and is recovered by the second recovery container 720 as a solution from each these PCBs have been extracted, i.e., a first analysis sample.

At this step, the alumina layer 250 can be heated from the outside of the pipe body 210. In the case of heating the alumina layer 250, the mono-ortho PCBs and the non-DL-PCBs can be efficiently extracted from the alumina layer 250 with a reduced usage of the extraction solvent. The temperature of heating the alumina layer 250 is normally preferably controlled to about 50°C to lower than the boiling temperature of the extraction solvent and specifically equal to or lower than 95°C.

At a subsequent step for preparing the analysis sample, after the second pump 542 has been temporarily stopped, the third valve 631 is switched such that the first ventilation path 630 and the open path 632 communicate with each other on the first extraction path 600, and the fourth valve 731 of the second extraction path 700 is switched to the closed state. Then, on the solvent outflow path 500, the second pump 542 is actuated again in a state in which the second valve 520 is maintained such that the flow path 510 communicates with the second solvent supply path 541. Accordingly, a moderate amount of solvent stored in the second solvent container 543 is supplied into the pipe body 210 through the opening 212 by way of the second solvent supply path 541 and the flow path 510.

The solvent supplied into the pipe body 210 from the flow path 510 passes through the alumina layer 250 and the adsorbent layer 240 in this order, and flows into the first branched path 215. The solvent is recovered by the first recovery container 620 through the first recovery path 610 of the first extraction path 600. At this point, the solvent dissolves the dioxin group, which includes the non-ortho PCBs, the PCDDs, and the PCDFs, having adsorbed to the adsorbent layer 240, and is recovered by the first recovery container 620 as a solution from which the dioxin group has been extracted, i.e., a second analysis sample.

At this step, the adsorbent layer 240 can be heated from the outside of the pipe body 210. In the case of heating the adsorbent layer 240, the dioxin group including the non-ortho PCBs, the PCDDs, and the PCDFs can be efficiently extracted from the adsorbent layer 240 with a reduced usage of the extraction solvent. The temperature of heating the adsorbent layer 240 is normally preferably controlled to about 50°C to lower than the boiling temperature of the extraction solvent and specifically equal to or higher than 80°C and equal to or lower than 95°C.

By the above-described extraction step, the analysis sample for the mono-ortho PCBs and the analysis sample for the non-ortho PCBs, the PCDDs, and the PCDFs are separately obtained.

These two types of analysis samples prepared as described above are separately applied to dioxin analysis. According to the type of solvent used for extracting the dioxins from the fractionating layer 230, a GC/MS method such as GC-HRMS, GC-MSMS, GC-QMS, or ion trap GC/MS, a gas chromatography method such as GC/ECD, or a bioassay method can be normally employed as the determination method.

In analysis of the analysis sample (the first analysis sample) for the mono-ortho PCBs, such an analysis sample substantially contains no dioxin group including the non-ortho PCBs, the PCDDs, and the PCDFs, and therefore, the mono-ortho PCBs can be quantified with a high accuracy without receiving influence of the dioxin group. Moreover, such an analysis sample contains the non-DL-PCBs together with the mono-ortho PCBs, and therefore, the non-DL-PCBs contained in the dioxin solution can be also quantified with a high accuracy. For example, according to food regulation standards (COMMISSION REGULATION (EU) No 1259/2011) in the European Union (EU), the dioxins and prescribed non-DL-PCBs (six types of PCBs of which IUPAC numbers are #28, #52, #101, #138, #153, and #180 and of which chlorine numbers are 3 to 7) are set as targets for analysis of harmful substances contained in food such as meats including beef and pork and eggs, but these PCBs can be quantified by analysis of this analysis sample.

On the other hand, in analysis of the analysis sample (the second analysis sample) for the non-ortho PCBs, the PCDDs, and the PCDFs, such an analysis sample substantially contain no mono-ortho PCBs and no non-DL-PCBs, and therefore, the non-ortho PCBs, the PCDDs, and the PCDFs can be quantified with a high accuracy without receiving influence of these PCBs.

Note that GC-MSMS or GC-TOFMS can be used as the GC/MS method, and in this case, two types of analysis samples can be used as a mixture for analysis at the same time.

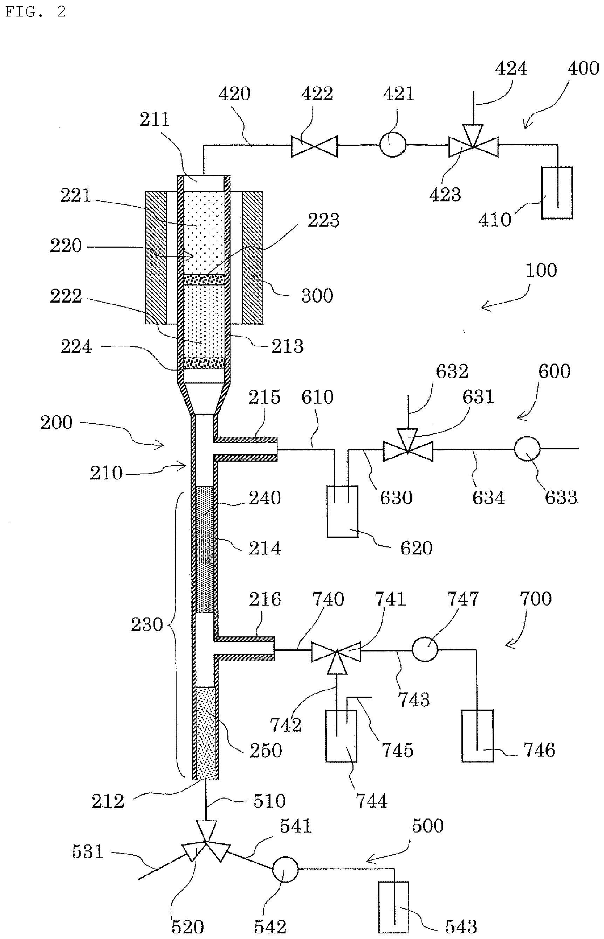

In the preparation apparatus 100, the second extraction path 700 can be changed as illustrated in Fig. 2. The changed second extraction path 700 has a solvent path 740 extending from the second branched path 216. The solvent path 740 air-tightly communicates, at one end thereof, with the second branched path 216, and at the other end, includes a fourth valve 741. The fourth valve 741 is a three-way valve, and is provided for making such switching that a solvent recovery path 742 and a third solvent supply path 743 communicate with each other and the solvent path 740 communicates with either one of the solvent recovery path 742 or the third solvent supply path 743.

The solvent recovery path 742 communicates with a second recovery container 744 for recovering a solvent. The second recovery container 744 has a ventilation pipe 745 allowing communication between the inside and the outside of the second recovery container 744. The third solvent supply path 743 has a third pump 747 communicating with a third solvent container 746 and provided for sending out a solvent stored in the third solvent container 746.

In this variation, the second solvent container 543 stores a solvent capable of extracting the dioxins (the mono-ortho PCBs and the non-DL-PCBs) adsorbing to the alumina layer 250, and the third solvent container 746 stores a solvent capable of extracting the dioxins (the non-ortho PCBs, the PCDDs, and the PCDFs) adsorbing to the adsorbent layer 240. The solvent stored in each of the containers 543, 746 can be selected according to the dioxin determination method.

Specifically, in the case of employing the gas chromatography method as the determination method, e.g., toluene or benzene can be used as the solvent stored in the third solvent container 746. Alternatively, a solvent mixture obtained in such a manner that an aliphatic hydrocarbon solvent or an organic chlorine-based solvent is added to the toluene or the benzene can be used. In the case of using the solvent mixture, the percentage of the toluene or the benzene is set to equal to or higher than 50% by weight. Examples of the aliphatic hydrocarbon solvent include n-pentane, n-hexane, n-heptane, n-octane, iso-octane, and cyclohexane. Moreover, examples of the organic chlorine-based solvent include dichloromethane, trichloromethane, and tetrachloromethane. Of these extraction solvents, the toluene is specifically preferred because the dioxins, specifically the non-ortho PCBs, the PCDDs, and the PCDFs, can be extracted by use of a small amount of the solvent. On the other hand, not only those similar to the solvent stored in the third solvent container 746 but also an organic chlorine-based solvent, a solvent mixture of an organic chlorine-based solvent and an aliphatic hydrocarbon solvent, and a solvent mixture obtained by addition of a small amount of toluene to an aliphatic hydrocarbon solvent can be used as the solvent stored in the second solvent container 543. However, the toluene is specifically preferred because the dioxins and the PCBs, specifically the mono-ortho PCBs and the non-DL-PCBs, can be extracted by use of a small amount of the solvent.

In the case of employing the bioassay method as the determination method, hydrophilic solvents such as dimethylsulfoxide (DMSO) and methanol can be used as the solvents stored in the second solvent container 543 and the third solvent container 746.

In the method for preparing the analysis sample for the dioxins by means of the preparation apparatus 100 with the changed second extraction path 700, the fourth valve 741 is, in an initial state, set such that the solvent path 740 communicates with the third solvent supply path 743. Then, after the dioxin fractionating step has been executed as described above, the dioxin extraction step is executed.

At the dioxin extraction step, after each layer of the purification layer 220 and the fractionating layer 230 has been dried as described above, the compressor 633 is stopped, and the fourth valve 741 is switched such that the solvent path 740 communicates with the solvent recovery path 742 on the second extraction path 700. Moreover, the second valve 520 is switched such that the flow path 510 communicates with the second solvent supply path 541 on the solvent outflow path 500, and the second pump 542 is actuated. Accordingly, a moderate amount of solvent stored in the second solvent container 543 is supplied into the pipe body 210 through the opening 212 by way of the second solvent supply path 541 and the flow path 510.

The solvent supplied into the pipe body 210 from the flow path 510 flows into the second branched path 216 through the alumina layer 250, and is recovered by the second recovery container 744 through the solvent path 740 of the second extraction path 700. At this point, the solvent dissolves the mono-ortho PCBs and the non-DL-PCBs adsorbing to the alumina layer 250, and such a solution with the PCBs, i.e., the first analysis sample, is recovered by the second recovery container 744.

At a subsequent step for preparing the analysis sample, after the second pump 542 has been stopped, the third valve 631 is switched such that the first ventilation path 630 and the open path 632 communicate with each other on the first extraction path 600, and the fourth valve 741 is switched such that the solvent path 740 communicates with the third solvent supply path 743 on the second extraction path 700. Then, the third pump 747 is actuated to supply a moderate amount of solvent stored in the third solvent container 746 into the pipe body 210 through the second branched path 216 by way of the third solvent supply path 743 and the solvent path 740.

The solvent supplied into the pipe body 210 through the second branched path 216 flows into the first branched path 215 through the adsorbent layer 240, and is recovered by the first recovery container 620 through the first recovery path 610 of the first extraction path 600. At this point, the solvent dissolves the dioxin group, which adsorbs to the adsorbent layer 240, including the non-ortho PCBs, the PCDDs, and the PCDFs, and such a solution with the dioxin group, i.e., the second analysis sample, is recovered by the first recovery container 620. The second analysis sample is prepared without the solvent passing through the alumina layer 250, and therefore, is fractionated from the mono-ortho PCBs and the non-DL-PCBs with a higher accuracy.

The obtained first analysis sample and the obtained second analysis sample are, as already described, applied to dioxin analysis.

In the preparation apparatus 100 configured such that the second extraction path 700 is changed as in Fig. 2, the order of extraction of the dioxin group including the non-ortho PCBs, the PCDDs, and the PCDFs from the adsorbent layer 240 and extraction of the mono-ortho PCBs and the non-DL-PCBs from the alumina layer 250 can be changed at the dioxin extraction step. That is, after the dioxin group including the non-ortho PCBs, the PCDDs, and the PCDFs has been first extracted from the adsorbent layer 240, the mono-ortho PCBs and the non-DL-PCBs can be extracted from the alumina layer 250.

### Second Embodiment

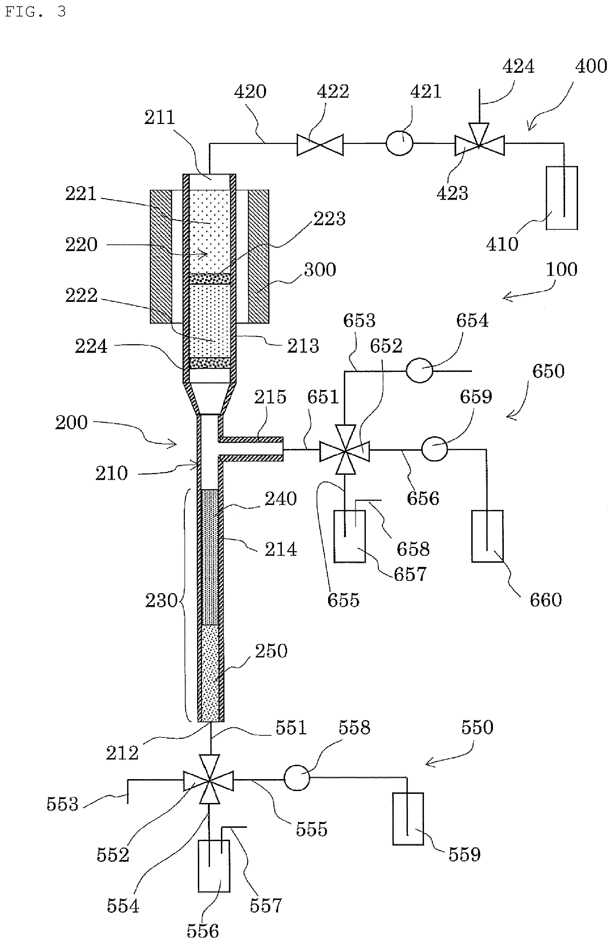

A second embodiment of an apparatus capable of performing an analysis sample preparation method according to the present invention will be described with reference to Fig. 3. In Fig. 3, a preparation apparatus 100 is capable of preparing an analysis sample suitable for analysis by a gas chromatography method, and mainly includes a fractionating tool 200, a heating apparatus 300, a solvent supply apparatus 400, a solvent outflow path 550, and an extraction path 650.

The fractionating tool 200 is different from the fractionating tool 200 described in the first embodiment in the structures of a small-diameter portion 214 and a fractionating layer 230 of a pipe body 210. Specifically, the small-diameter portion 214 has only a first branched path 215 as a branched path. The fractionating layer 230 is configured such that an adsorbent layer 240 and an alumina layer 250 are in close contact with each other. Thus, the small-diameter portion 214 has a shorter length than that of the fractionating tool 200 described in the first embodiment.

The heating apparatus 300 and the solvent supply apparatus 400 are configured as described in the first embodiment.

The solvent outflow path 550 has a flow path 551 air-tightly connected to an opening 212 of the pipe body 210. The flow path 551 has a second valve 552. The second valve 552 is a four-way valve. The second valve 552 communicates with a discarding path 553 for discarding a solvent from the pipe body 210, a recovery path 554 for recovering a solvent from the pipe body 210, and a supply path 555 for supplying a solvent to the pipe body 210, and makes such switching that the flow path 551 communicates with any one of the discarding path 553, the recovery path 554, or the supply path 555.

The recovery path 554 has a solvent recovery container 556, and the recovery container 556 has a ventilation pipe 557 allowing communication between the inside and the outside of the recovery container 556. The supply path 555 has a second pump 558, and communicates with a second solvent container 559 for storing a dioxin extraction solvent trapped by the fractionating tool 200.

The extraction solvent stored in the second solvent container 559 can dissolve dioxins, and toluene or benzene can be used. Alternatively, a solvent mixture obtained in such a manner that an aliphatic hydrocarbon solvent or an organic chlorine-based solvent is added to the toluene or the benzene can be used. In the case of using the solvent mixture, the percentage of the toluene or the benzene is set to equal to or higher than 50% by weight. Examples of the aliphatic hydrocarbon solvent used for such a solvent mixture include n-pentane, n-hexane, n-heptane, n-octane, iso-octane, and cyclohexane. Moreover, examples of the organic chlorine-based solvent include dichloromethane, trichloromethane, and tetrachloromethane. Of these extraction solvents, the toluene is specifically preferred because the dioxins can be extracted from the fractionating tool 200 by use of a small amount of the solvent.

The extraction path 650 has a solvent path 651 extending from the first branched path 215. The solvent path 651 air-tightly communicates, at one end thereof, the first branched path 215, and at the other end, includes a third valve 652. The third valve 652 is a three-way valve. The third valve 652 communicates with an air supply path 653 including a compressor 654 for sending compressed air, a recovery path 655 for recovering a solvent from the first branched path 215, and a supply path 656 for supplying a solvent to the pipe body 210, and makes such switching that the solvent path 651 communicates with any one of the air supply path 653, the recovery path 655, or the supply path 656.

The recovery path 655 has a recovery container 657 for recovering a solvent, and the recovery container 657 has a ventilation pipe 658 allowing communication between the inside and the outside of the recovery container 657. The supply path 656 has a third pump 659, and communicates with a third solvent container 660 for storing a dioxin extraction solvent trapped by the fractionating tool 200.

The extraction solvent stored in the third solvent container 660 does not substantially dissolve a dioxin group including non-ortho PCBs, PCDDs, and PCDFs, and exhibits excellent mono-ortho PCBs and non-DL-PCBs dissolubility. For example, an organic chlorine-based solvent, a solvent mixture obtained by addition of an aliphatic hydrocarbon solvent to an organic chlorine-based solvent, or a solvent mixture (a toluene content is normally about 10 to 15% by weight) obtained by addition of toluene to an aliphatic hydrocarbon solvent. The organic chlorine-based solvent used herein is, for example, dichloromethane, trichloromethane, and tetrachloromethane. Moreover, the aliphatic hydrocarbon solvent is, for example, n-pentane, n-hexane, n-heptane, n-octane, iso-octane, or cyclohexane.

Next, the method for preparing the dioxin analysis sample by means of the above-described preparation apparatus 100 will be described. First, in the preparation apparatus 100, a first valve 422, an air introduction valve 423, the second valve 552, and the third valve 652 are set to prescribed initial states. That is, the first valve 422 is set to an open state, and the air introduction valve 423 is set to communicate with a first solvent container 410 side. Moreover, the second valve 552 is set such that the flow path 551 communicates with the discarding path 553. Further, the third valve 652 is set such that the solvent path 651 communicates with the air supply path 653.

Next, after a dioxin fractionating step has been executed as in the first embodiment, each layer of a purification layer 220 and the fractionating layer 230 is dried, and a dioxin extraction step is executed. The treatment for drying the purification layer 220 can be executed as in the first embodiment. In the subsequent treatment for drying the fractionating layer 230, the first valve 422 of the solvent supply apparatus 400 is switched to a closed state. Then, on the extraction path 650, the compressor 654 is actuated.

By actuation of the compressor 654, compressed air is supplied to the first branched path 215 through the air supply path 653 and the solvent path 651. Such compressed air flows into the flow path 551 through the opening 212 by way of the fractionating layer 230, and is discharged through the discarding path 553. At this point, a solvent remaining in each layer of the fractionating layer 230 is pushed out by the compressed air, and is discharged from the discarding path 553 together with the compressed air. As a result, each layer of the fractionating layer 230 is dried.

At the dioxin extraction step, the second valve 552 is first switched such that the flow path 551 communicates with the recovery path 554 on the solvent outflow path 550. Moreover, on the extraction path 650, the third valve 652 is switched such that the solvent path 651 communicates with the supply path 656, and the third pump 659 is actuated. Accordingly, a moderate amount of solvent stored in the third solvent container 660 is supplied into the pipe body 210 through the first branched path 215 by way of the supply path 656 and the solvent path 651.

The solvent supplied into the pipe body 210 through the first branched path 215 passes through the fractionating layer 230, and flows into the flow path 551 and the recovery path 554 through the opening 212. The solvent is recovered by the recovery container 556. At this point, the solvent dissolves and extracts the mono-ortho PCBs and the non-DL-PCBs adsorbing to the alumina layer 250, and such a solution with the PCBs, i.e., a first analysis sample, is recovered by the recovery container 556.

At a subsequent step for extracting the dioxins, the third pump 659 is stopped, and the third valve 652 is switched such that the solvent path 651 communicates with the recovery path 655 on the extraction path 650. Then, on the solvent outflow path 550, the second valve 552 is switched such that the flow path 551 communicates with the supply path 555, and the second pump 558 is actuated. Accordingly, a moderate amount of solvent stored in the second solvent container 559 is supplied into the pipe body 210 through the opening 212 by way of the supply path 555 and the flow path 551.

The solvent supplied into the pipe body 210 through the flow path 551 passes through the alumina layer 250 and the adsorbent layer 240 in this order, and flows into the first branched path 215. The solvent is recovered by the recovery container 657 through the solvent path 651 and the recovery path 655 of the extraction path 650. At this point, the solvent dissolves and extracts the dioxin group, which adsorbs to the adsorbent layer 240, including the non-ortho PCBs, the PCDDs, and the PCDFs, and such a solution with the dioxin group, i.e., a second analysis sample, is recovered by the recovery container 657.

By the above-described steps, the analysis sample for the mono-ortho PCBs and the analysis sample for the non-ortho PCBs, the PCDDs, and the PCDFs are separately obtained, and each analysis sample is applied to analysis by the gas chromatography method.

### Third Embodiment

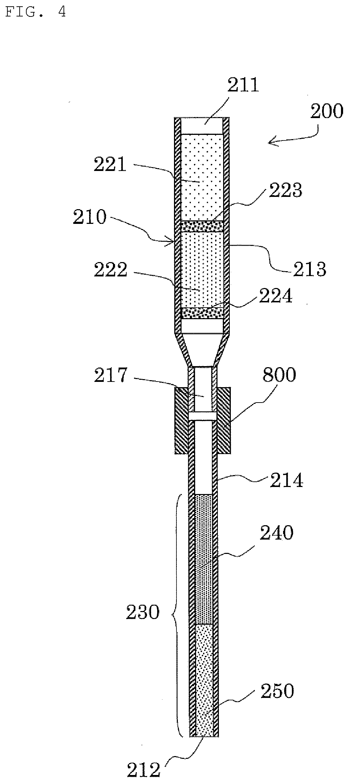

Another example of a fractionating tool capable of performing an analysis sample preparation method according to the present invention will be described with reference to Fig. 4. In the figure, a fractionating tool 200 includes a pipe body 210 having a large-diameter portion 213 and a small-diameter portion 214 as in the fractionating tool 200 used in the preparation apparatus 100 of the second embodiment. However, the fractionating tool 200 is divided into the large-diameter portion 213 and the small-diameter portion 214, and the large-diameter portion 213 and the small-diameter portion 214 are detachably coupled to each other through a coupling tool 800 to form the continuous pipe body 210.

The large-diameter portion 213 is formed in a cylindrical shape opening at both ends, and at an end portion on a sulfuric silica gel layer 222 side, has a neck portion 217 having the same outer and inner diameters as those of the small-diameter portion 214. The small-diameter portion 214 is formed in a cylindrical shape opening at both ends, and an adsorbent layer 240 and an alumina layer 250 are in close contact with each other in a fractionating layer 230. The coupling tool 800 is, for example, formed in a cylindrical shape with a resin material having resistance to various organic solvents, specifically a hydrocarbon solvent, or other materials, and the large-diameter portion 213 and the small-diameter portion 214 are liquid-tightly coupled to each other in such a manner that the neck portion 217 of the large-diameter portion 213 and an end portion of the small-diameter portion 214 on an adsorbent layer 240 side are inserted into the coupling tool 800.

When a dioxin analysis sample is prepared using the preparation apparatus 100 of this example, a dioxin fractionating step is, as in the first embodiment, executed in a state in which the large-diameter portion 213 and the small-diameter portion 214 are coupled to each other in the fractionating tool 200. The fractionating step can be executed by manual operation. Then, after a purification layer 220 and the fractionating layer 230 have been dried subsequently to the fractionating step, the small-diameter portion 214 is separated from the coupling tool 800.

In extraction of dioxins from the fractionating layer 230, a solvent not substantially dissolving a dioxin group including non-ortho PCBs, PCDDs, and PCDFs and exhibiting excellent mono-ortho PCBs and non-DL-PCBs dissolubility is, as in the case of the second embodiment, supplied from the end portion of the small-diameter portion 214 on the adsorbent layer 240 side. In this manner, mono-ortho PCBs and non-DL-PCBs adsorbing to the alumina layer 250 are extracted, and a first analysis sample is obtained. Thereafter, a solvent capable of dissolving the dioxins is supplied from an end portion (an opening 212) of the small-diameter portion 214 on an alumina layer 250 side. In this manner, the dioxin group, which adsorbs to the adsorbent layer 240, including the non-ortho PCBs, the PCDDs, and the PCDFs is extracted, and a second analysis sample is obtained.

Such extraction operation can be executed by manual operation, but can be mechanically executed.

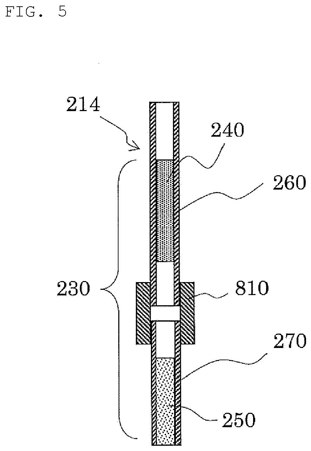

Part of a variation of the fractionating tool 200 of this example is illustrated in Fig. 5. The small-diameter portion 214 of the fractionating tool 200 according to this variation is divided into a first portion 260 filled with the adsorbent layer 240 and a second portion 270 filled with the alumina layer 250, and the first portion 260 and the second portion 270 are detachably coupled to and integrated with each other by a coupling tool 810. The coupling tool 810 is similar to the coupling tool 800 for coupling the large-diameter portion 213 and the small-diameter portion 214.

The fractionating tool 200 of this variation is configured so that the small-diameter portion 214 can be separated from the large-diameter portion 213 and the small-diameter portion 214 can be further separated into the first portion 260 and the second portion 270. Thus, when the dioxins are extracted from the fractionating layer 230, the operation of extracting the dioxins and the like can be separately executed for the adsorbent layer 240 of the first portion 260 and the alumina layer 250 of the second portion 270, and the analysis sample for the mono-ortho PCBs and the non-DL-PCBs and the analysis sample for the dioxin group including the non-ortho PCBs, the PCDDs, and the PCDFs can be fractionated with a higher accuracy.

### Other Embodiments

(1) The fractionating tool 200 described above in each embodiment is configured such that the silver nitrate silica gel layer 221 is arranged on the opening 211 side in the purification layer 220, but the order of the silver nitrate silica gel layer 221 and the sulfuric silica gel layer 222 can be changed.

Note that in a case where the silver nitrate silica gel layer 221 and the sulfuric silica gel layer 222 are interchanged, there is a probability that the non-DL-PCBs with smaller chlorine numbers react with the sulfuric silica gel layer 222 and the rate of recovery of the non-DL-PCBs with smaller chlorine numbers is lowered in the analysis sample. For this reason, in a case where not only the dioxins but also the non-DL-PCBs with smaller chlorine numbers need to be analyzed (e.g., a case where dioxins in food are analyzed according to the food regulation standards in the EU), the silver nitrate silica gel layer 221 is preferably arranged on the opening 211 side in the purification layer 220.

(2) The fractionating tool 200 described above in each embodiment may be configured such that the first active silica gel layer 223 and the second active silica gel layer 224 are omitted from the purification layer 220.

(3) The large-diameter portion 213 of the fractionating tool 200 can be divided into a portion filled with the silver nitrate silica gel layer 221 and a portion filled with the sulfuric silica gel layer 222, and these portions can be coupled to each other upon use. With this configuration, denaturalization of one of the silver nitrate silica gel layer 221 or the sulfuric silica gel layer 222 due to influence of the other one of the silver nitrate silica gel layer 221 or the sulfuric silica gel layer 222 can be reduced. As a result, the capacity of the purification layer 220 for purifying the dioxin solution is less likely to be degraded, and therefore, there is a probability that the dioxin recovery rate can be enhanced.

(4) In the dioxin analysis sample preparation method according to each of the above-described embodiments, the purification layer 220 is heated by the heating apparatus 300, but the preparation method according to each embodiment can be similarly performed even in a case where the purification layer 220 is not heated.

(5) In the dioxin analysis sample preparation method according to each of the above-described embodiments, the treatment for drying the purification layer 220 and the fractionating layer 230 can be changed as necessary by any of an air suction method and a compressed air supply method using a compressor. Moreover, the purification layer 220 and the fractionating layer 230 can be dried by a nitrogen gas supply. Further, the treatment for drying the purification layer 220 and the fractionating layer 230 can be omitted.

### Examples

Hereinafter, the present invention will be specifically described with reference to Examples and the like, but is not limited to these Examples and the like.

### Dioxin Solution

A dioxin solution used in the following Examples and the like is as follows.

### Standard Solution:

A standard dioxin substance (a product name "DF-LCS-A" of Wellington Laboratories Inc.), which has a known concentration, of 1 mL and a standard PCBs substance (a product name "PCB-LCS-H" of Wellington Laboratories Inc.), which has a known concentration, of 1 mL were added to and dissolved in n-hexane of 100 mL, and the resultant was taken as a standard dioxin solution. The standard dioxin substance includes PCDDs, PCDFs, and DL-PCBs labelled by 13C12. The standard PCBs substance includes seven types of non-DL-PCBs which are labelled by 13C12 and of which IUPAC numbers are #28, #52, #101, #138, #153, #170, and #180. Of these substances, six types of PCBs isomers (PCBs isomers with chlorine numbers of 3 to 7) with #28, #52, #101, #138, #153 and #180 are targeted for food regulations in the EU.

### Environmental Sample Solution A:

A Soxhlet extraction method using toluene as an extraction solvent was applied to fly ash, which had been collected from a general waste incineration facility, of 0.3 g, and in this manner, a toluene extract was obtained. The toluene was removed from the toluene extract, and the residue thereof was dissolved in n-hexane. In this manner, a hexane solution of 2 mL was prepared. A standard solution of 0.02 mL was added to the total amount of the hexane solution, and the resultant was taken as an environmental sample solution A.

### Environmental Sample Solution B:

Operation was performed as in preparation of the environmental sample solution A, except that soil, which had been collected from a factory site, of 5 g was used instead of fly ash of 0.3 g. The resultant solution was taken as an environmental sample solution B.

### Environmental Sample Solution C:

Operation was performed as in preparation of the environmental sample solution A, except that a bottom material, which had been collected from a river, of 5 g was used instead of fly ash of 0.3 g. The resultant solution was taken as an environmental sample solution C.

### Environmental Sample Solution D:

Operation was performed as in preparation of the environmental sample solution A, except that sludge, which had been collected from a sewage treatment facility, of 5 g was used instead of fly ash of 0.3 g. The resultant solution was taken as an environmental sample solution D.

### Simulant Environmental Sample Solution:

Toluene of 100 µL was added to a standard solution of 0.02 mL, and the resultant is taken as a simulant environmental sample solution.

### Food Sample Solution A:

A standard solution of 0.02 mL was added to sunflower oil (a product name "S5007" of Merck) of 3g, and the resultant was taken as a food sample solution A.

### Food Sample Solution B:

Operation was performed as in preparation of the environmental sample solution A, except that an oil content, which had been extracted using an organic solvent from freeze-dried pork, of 3 g was used instead of fly ash of 0.3 g. The resultant solution was taken as a food sample solution B.

### Filler

A filler used in a fractionating tool in the Examples and the like below is as follows.

### Silver Nitrate Silica Gel:

The total amount of an aqueous solution obtained in such a manner that silver nitrate (manufactured by Wako Pure Chemical Corporation) of 11.2g is dissolved in distilled water of 30 mL was added to active silica gel (manufactured by Kanto Chemical Co., Inc.) of 100 g, and was uniformly mixed. Silver nitrate silica gel was used, which was prepared in such a manner that the resultant active silica gel is heated to 70°C under a reduced pressure by means of a rotary evaporator and is dried.

### Sulfuric Silica Gel:

Concentrated sulfuric acid (manufactured by Wako Pure Chemical Corporation) of 78.7 g was uniformly added to active silica gel (manufactured by Kanto Chemical Co., Inc.) of 100 g. Thereafter, the resultant was dried to prepare sulfuric silica gel.

### Activated Carbon-Containing Silica Gel:

Activated carbon-containing silica gel was used, which was obtained in such a manner that activated carbon (a product name "Kuraray Coal PK-DN" of Kuraray Co., Ltd.) is added to active silica gel (manufactured by Kanto Chemical Co., Inc.) and is uniformly mixed. The content of the activated carbon was set to 0.018% by weight or 3.0% by weight.

### Graphite-Containing Silica Gel:

Graphite-containing silica gel was used, which was obtained in such a manner that graphite (a product name "ENVI-Carb" of Sigma-Aldrich) is added to active silica gel (manufactured by Kanto Chemical Co., Inc.) and is uniformly mixed. The content of the graphite was set to 12.5% by weight.

### Graphite:

A product name "ENVI-Carb" of Sigma-Aldrich was used.

### Magnesium Silicate:

A product name "Florisil, 75 to 150 µm" of Fujifilm Wako Pure Chemical Corporation was used.

### Active Magnesium Silicate:

Magnesium silicate (a product name "Florisil, 75 to 150 µm" of Fujifilm Wako Pure Chemical Corporation) activated by heating for two hours under a nitrogen air flow set to a flow rate of 0.5 to 1.0 L/minute in a tubular furnace was used. A heating treatment temperature was as shown in Table 1 described later.

### Graphite-Mixed Active Magnesium Silicate A:

Graphite ("ENVI-Carb" of Sigma-Aldrich) was added to magnesium silicate (a product name "Florisil, 75 to 150 µm" of Fujifilm Wako Pure Chemical Corporation), and was uniformly mixed. In this manner, a mixture was prepared. The resultant from heating of the mixture for two hours in a nitrogen air flow set to a flow rate of 0.5 to 1.0 L/minute in a tubular furnace was used. The content of the graphite in the mixture and the temperature of heating of the mixture are as shown in Table 1 described later.

### Graphite-Mixed Active Magnesium Silicate B:

Graphite ("ENVI-Carb" of Sigma-Aldrich) heated for two hours in a nitrogen air flow set to a flow rate of 0.5 to 1.0 L/minute in a tubular furnace set to 450°C was added to magnesium silicate (a product name "Florisil, 75 to 150 µm" of Fujifilm Wako Pure Chemical Corporation) separately activated by heating for two hours in a nitrogen air flow set to a flow rate of 0.5 to 1.0 L/minute in a tubular furnace, and was uniformly mixed. The resultant mixture was used. The temperature of heating of the magnesium silicate and the content of the graphite are as shown in table 1 described later.

### Alumina:

A product name "Aluminum Oxide 90 active basic - (activity stage I) for column chromatography" (a particle size of 0.063 to 0.200 mm) of Merck was used.

### Examples 1 to 16 and Comparative Examples 1 to 6

An analysis sample was prepared from a dioxin solution by means of the dioxin analysis sample preparation apparatus illustrated in Fig. 1. The specifications of the fractionating tool used in the preparation apparatus are as follows.

### Purification Layer:

In a large-diameter portion of a pipe body set to an outer diameter of 18.5 mm, an inner diameter of 12.5 mm, and a length of 200 mm, silver nitrate silica gel of 4.4 g (a filling height of 60 mm) was stacked on sulfuric silica gel of 8.5 g (a filling height of 80 mm) to form a purification layer as illustrated in Fig. 1 (a stack of a first active silica gel layer and a second active silica gel layer is omitted).

### Fractionating Layer:

In a small-diameter portion of the pipe body set to an outer diameter of 8 mm, an inner diameter of 6 mm, and a length of 100 mm, an adsorbent layer and an alumina layer was formed as illustrated in Fig. 1. Each of the adsorbent layer and the alumina layer is formed by filling of a material shown in Table 1. Table 1 shows Comparative Examples corresponding to Examples.

[Table 1]

**Table 1**

| | Adsorbent layer | | | | | | Alumina Layer | | | Dioxin Solution |
|---|---|---|---|---|---|---|---|---|---|---|
| | Filler | | Heating Treatment Temperature (°C) | Graphite Mixing Timing | Filing Amount (g) | Filling Height (mm) | Filler | Filling Amount (g) | Filling Height (mm) | |
| | Type | Graphite Content (% by weight) | | | | | | | | |
| Example 1 | Active Magnesium Silicate | 0 | 450 | - | 0.45 | 30 | Alumina | 0.755 | 32.5 | Standard Solution |
| Comparative Example 1 | Active Magnesium Silicate | 0 | 700 | - | 0.45 | 30 | Alumina | 0.755 | 32.5 | Standard Solution |
| Comparative Example 2 | Active Magnesium Silicate | 0 | 800 | - | 0.52 | 30 | Alumina | 0.755 | 32.5 | Standard Solution |
| Comparative Example 3 | Active Magnesium Silicate | 0 | 900 | - | 0.52 | 30 | Alumina | 0.755 | 32.5 | Standard Solution |
| Comparative Example 4 | Magnesium Silicate | 0 | - | - | 0.45 | 30 | Alumina | 0.755 | 32.5 | Standard Solution |
| Example 2 | Graphite-Mixed Active Magnesium Silicate A | 12.5 | 300 | Before Heating | 0.415 | 30 | Alumina | 0.755 | 32.5 | Standard Solution |
| Example 3 | Graphite-Mixed Active Magnesium Silicate A | 12.5 | 500 | Before Heating | 0.415 | 30 | Alumina | 0.755 | 32.5 | Standard Solution |
| Example 4 | Graphite-Mixed Active Magnesium Silicate A | 12.5 | 650 | Before Heating | 0.415 | 30 | Alumina | 0.755 | 32.5 | Standard Solution |
| Comparative Example 5 | Graphite-Mixed Active Magnesium Silicate A | 12.5 | 700 | Before Heating | 0.415 | 30 | Alumina | 0.755 | 32.5 | Standard Solution |
| Example 5 | Graphite-Mixed Active Magnesium Silicate A | 6 | 450 | Before Heating | 0.415 | 30 | Alumina | 0.755 | 32.5 | Standard Solution |
| Example 6 | Graphite-Mixed Active Magnesium Silicate A | 12.5 | 450 | Before Heating | 0.415 | 30 | Alumina | 0.755 | 32.5 | Standard Solution |
| Example 7 | Graphite-Mixed Active Magnesium Silicate A | 25 | 450 | Before Heating | 0.415 | 30 | Alumina | 0.755 | 32.5 | Standard Solution |
| Example 8 | Graphite-Mixed Active Magnesium Silicate A | 12.5 | 450 | Before Heating | 0.415 | 30 | Alumina | 0.755 | 32.5 | Environmental Sample Solution B |
| Example 9 | Graphite-Mixed Active Magnesium Silicate A | 12.5 | 450 | Before Heating | 0.415 | 30 | Alumina | 0.755 | 32.5 | Environmental Sample Solution C |
| Example 10 | Graphite-Mixed Active Magnesium Silicate A | 12.5 | 450 | Before Heating | 0.415 | 30 | Alumina | 0.755 | 32.5 | Environmental Sample Solution A |
| Example 11 | Graphite-Mixed Active Magnesium Silicate A | 12.5 | 450 | Before Heating | 0.415 | 30 | Alumina | 0.755 | 32.5 | Environmental Sample Solution D |
| Example 12 | Graphite-Mixed Active Magnesium Silicate A | 12.5 | 450 | Before Heating | 0.415 | 30 | Alumina | 0.755 | 32.5 | Simulant Environmental Sample Solution |
| Example 13 | Graphite-Mixed Active Magnesium Silicate A | 12.5 | 450 | Before Heating | 0.415 | 30 | Alumina | 0.755 | 32.5 | Food Sample Solution A |
| Example 14 | Graphite-Mixed Active Magnesium Silicate A | 12.5 | 450 | Before Heating | 0.415 | 30 | Alumina | 0.755 | 32.5 | Food Sample Solution B |
| Example 15 | Graphite-Mixed Active Magnesium Silicate B | 12.5 | 450 (*) | After Heating | 0.415 | 30 | Alumina | 0.755 | 32.5 | Standard Solution |
| Example 16 | Graphite-Mixed Active Magnesium Silicate B | 12.5 | 500 (*) | After Heating | 0.415 | 30 | Alumina | 0.755 | 32.5 | Environmental Sample Solution A |
| Comparative Example 6 | Graphite | 100 | - | - | 0.61 | 30 | Alumina | 0.755 | 32.5 | Standard Solution |

| | | | | | | | | | | |
|---|---|---|---|---|---|---|---|---|---|---|
| (*) Magnesium Silicate Heating Treatment Temperature before Mixing with Graphite | | | | | | | | | | |

In dioxin analysis sample preparation operation, a dioxin solution of about 4 mL was added to the silver nitrate silica gel layer of the purification layer, and the purification layer was heated to 60°C. Then, n-hexane of 90 mL was gradually supplied to the purification layer such that the n-hexane passes through the purification layer and the fractionating layer. After the n-hexane has passed through the fractionating layer, compressed air passes through the fractionating layer to dry the fractionating layer. Then, the alumina layer in the fractionating layer was heated to 90°C, toluene of 1.0 mL was supplied to the alumina layer from an opening side at a lower end of the pipe body, and the toluene having passed through the alumina layer was recovered through a second branched path. In this manner, a first analysis sample was obtained. Next, the adsorbent layer in the fractionating layer was heated to 90°C, toluene of 1.5 mL was supplied to the adsorbent layer through the alumina layer from the opening side at the lower end of the pipe body, and the toluene having passed through the adsorbent layer was recovered through a first branched path. In this manner, a second analysis sample was obtained. Time required until the second analysis sample is obtained after addition of the dioxin solution was about two hours in any of Examples and Comparative Examples.

Each of the first analysis sample and the second analysis sample was separately quantitatively analyzed by a HRGC/HRMS method, and the rate of recovery of dioxins and non-DL-PCBs was calculated. The dioxin recovery rate indicates the percentage (%) of the amount of dioxins contained in the analysis sample with respect to the amount of dioxins contained in the dioxin solution added to the purification layer. The same also applies to the rate of recovery of the non-DL-PCBs. Results are shown in Tables 2 to 7. In Tables 2 to 7, "-" indicates that no recovery rate was calculated.

### [Table 2]

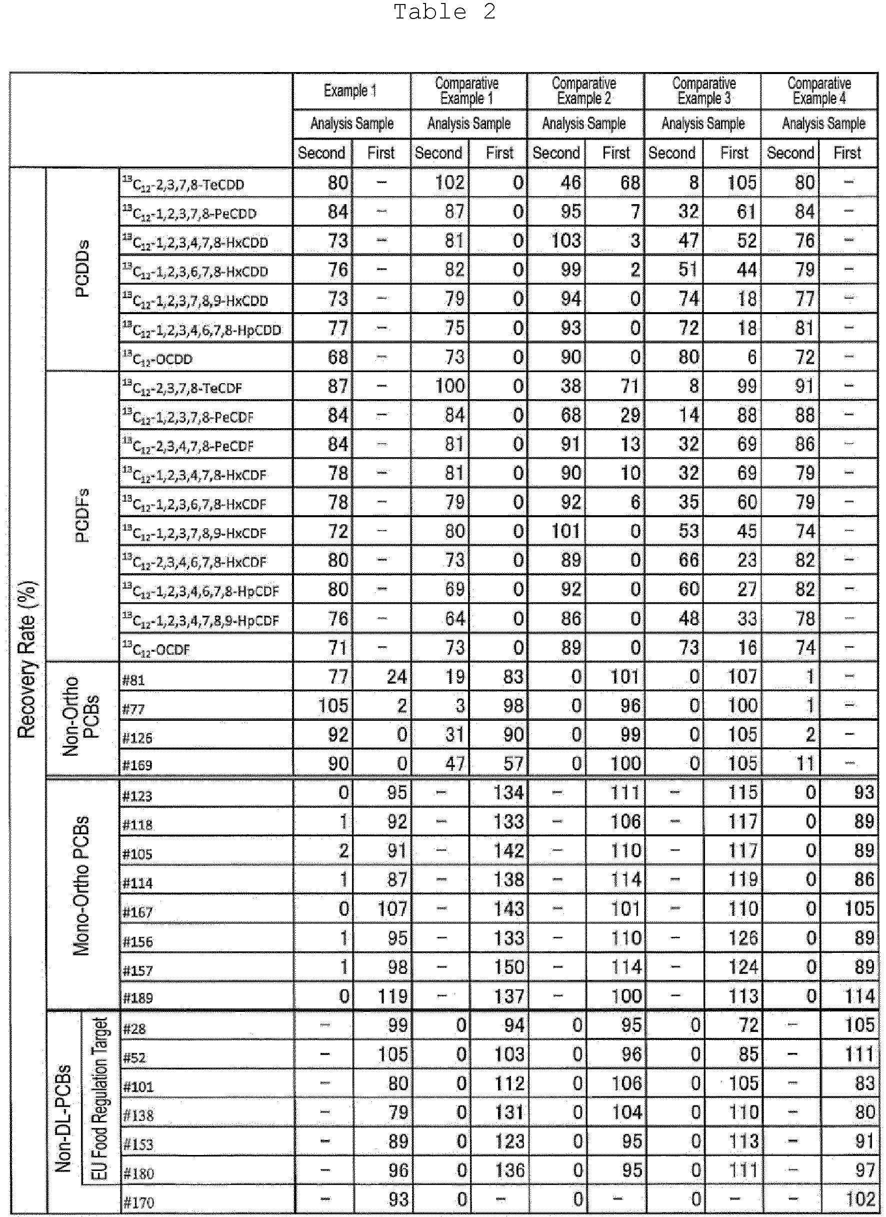

### [Table 3]

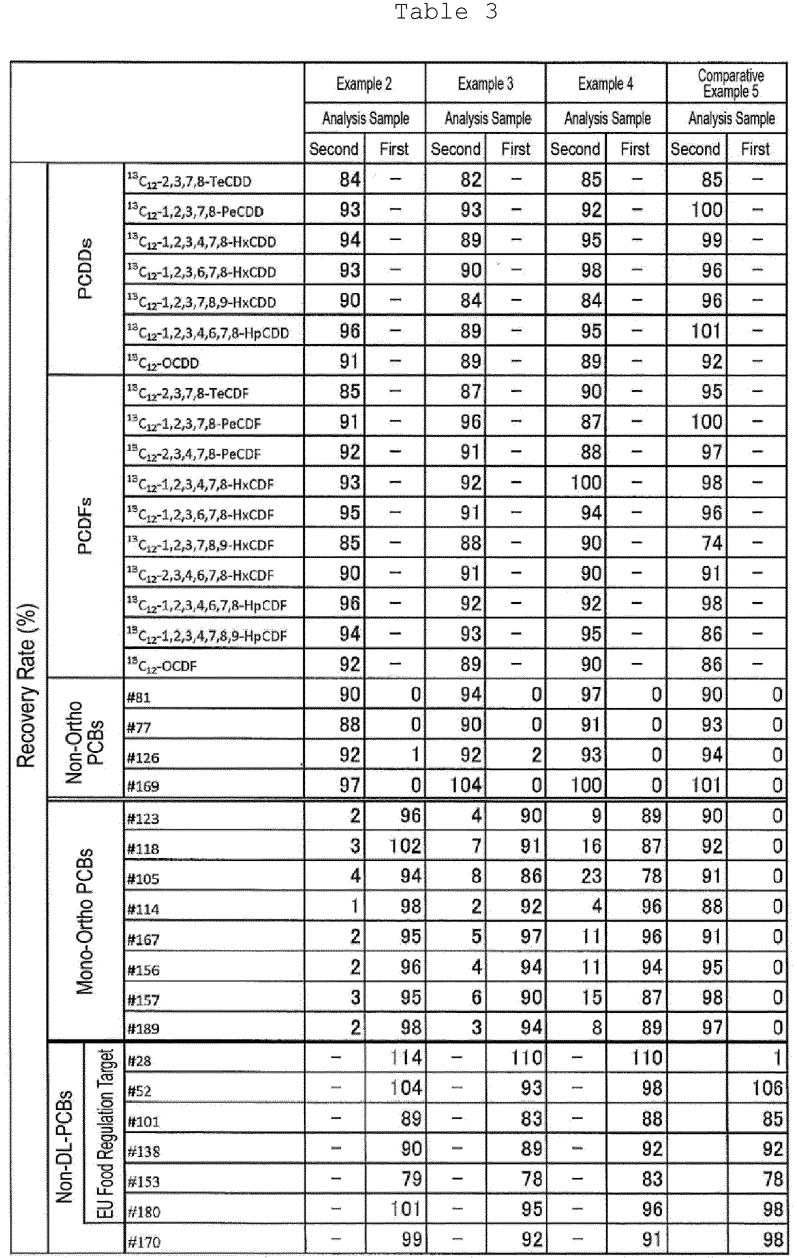

### [Table 4]

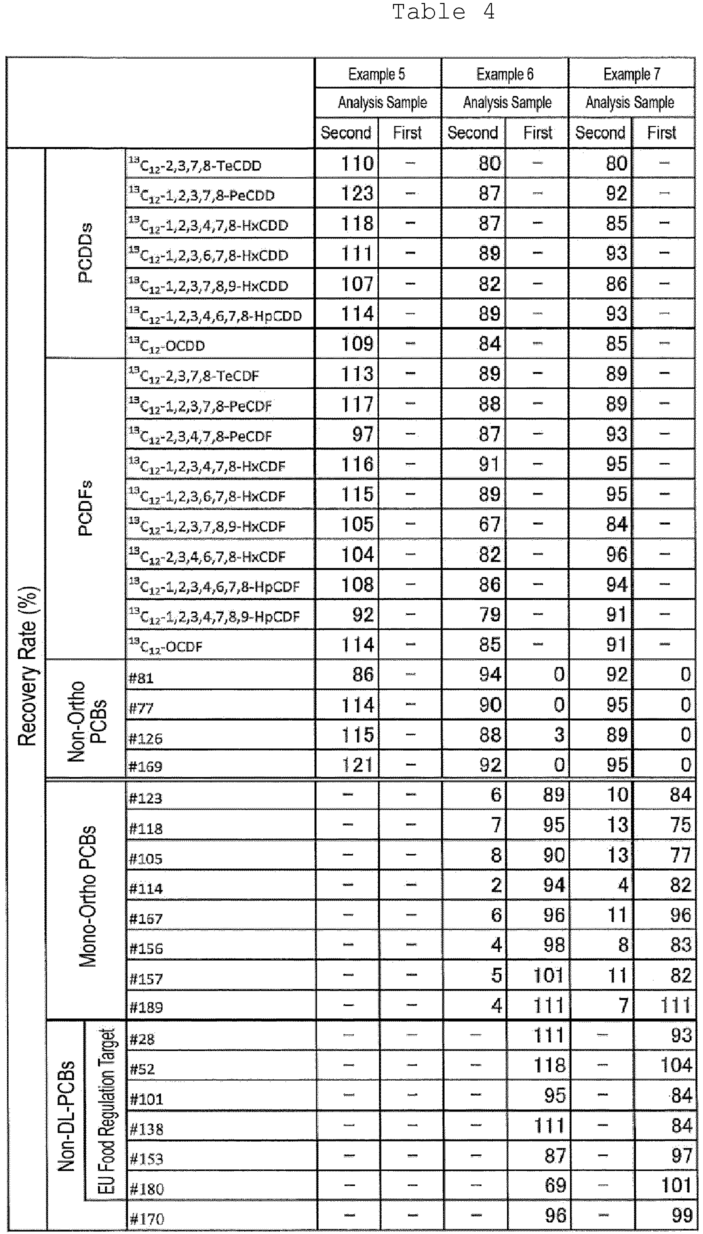

### [Table 5]

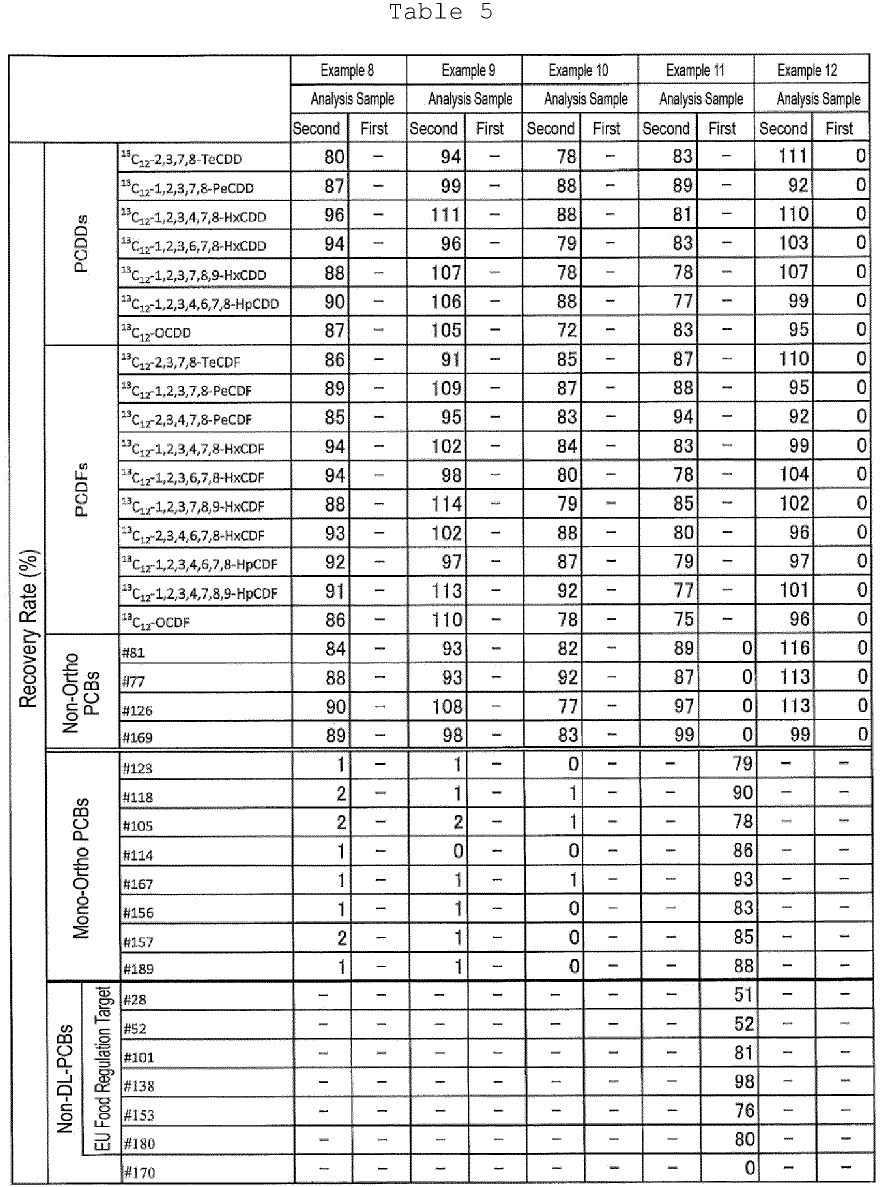

### [Table 6]

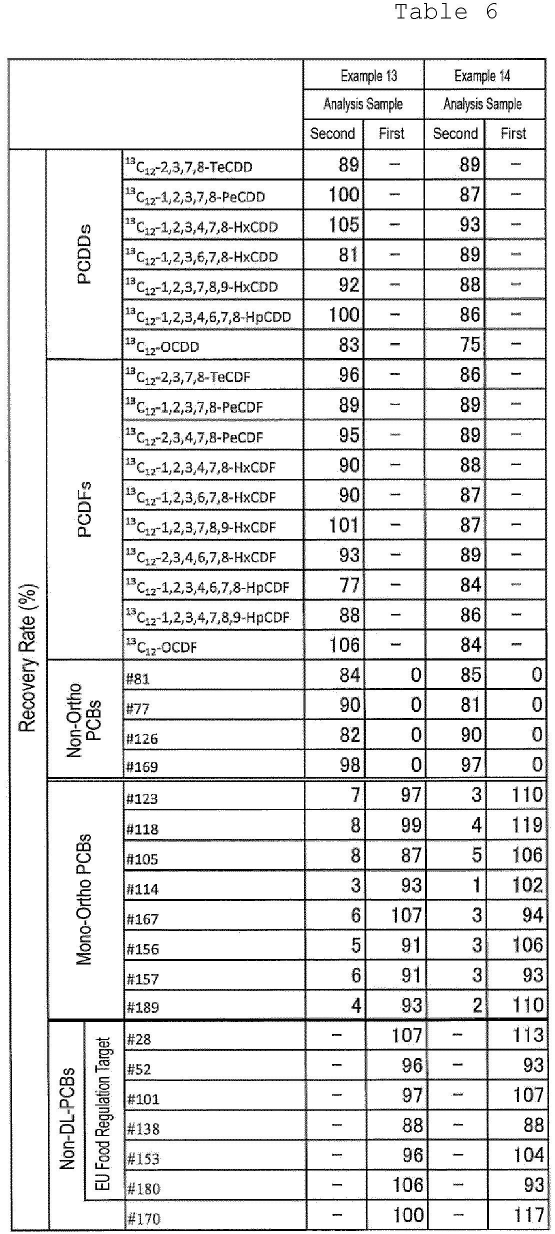

### [Table 7]

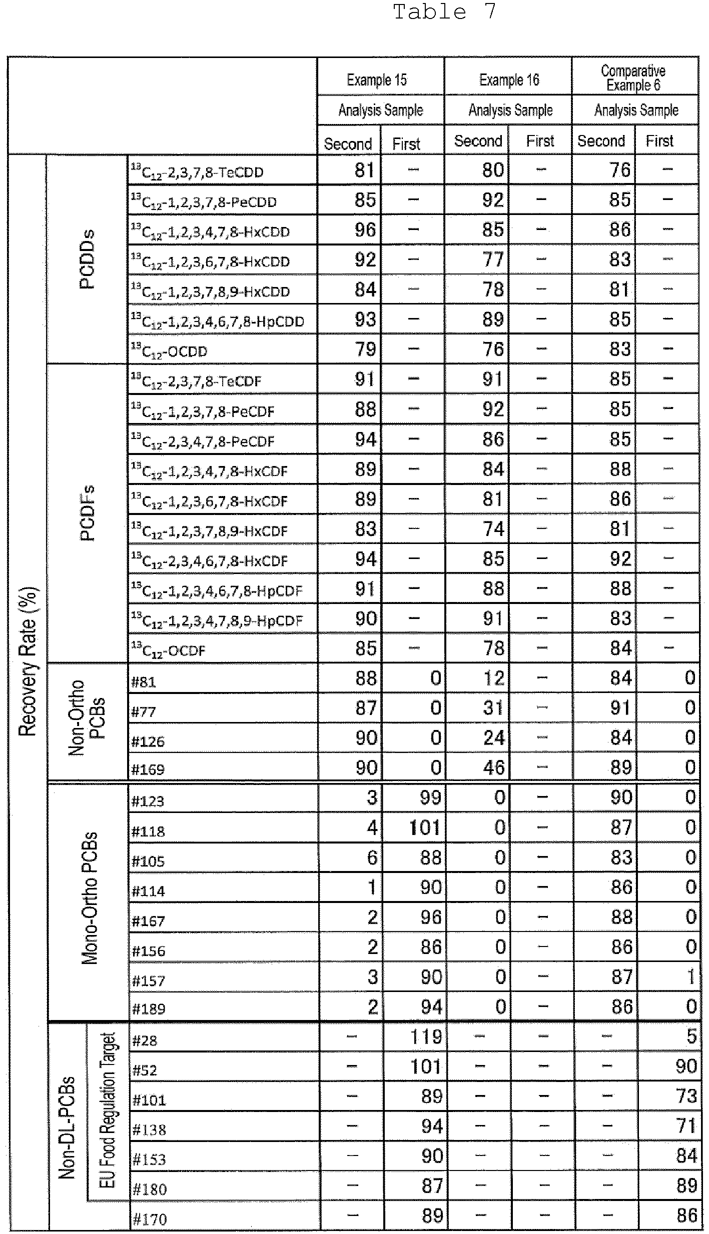

Regarding the dioxin solutions used in Examples 1 to 15, it is shown that each dioxin contained therein was recovered within a range of 60 to 120% as the regulation standards (COMMISSION REGULATION (EU) No 709/2014) in the European Union (EU) and is fractionated into a dioxin group including the non-ortho PCBs, the PCDDs, and the PCDFs and the mono-ortho PCBs with a high accuracy. In Examples 13 and 14 where the dioxin solutions are those derived from food (the food sample solutions A, B), the rate of recovery of the non-DL-PCBs targeted for the food regulations in the EU is high.

In Example 16, there is a defect in the non-ortho PCBs recovery rate of the second analysis sample. It is assumed that this is because the dioxin solution is a solution (the environmental sample solution A) containing impurity components in an environmental sample (the fly ash) and influence of these impurity components, specifically an aromatic hydrocarbon compound, is provided. On the other hand, in Examples 8 to 12, the first analysis sample and the second analysis sample were prepared from a dioxin solution (the environmental sample solutions A to D and the simulant environmental sample solution) containing impurity components in an environmental sample as in Example 16, but the graphite-mixed active magnesium silicate used in the adsorbent layer was one obtained by heating of the mixture of the magnesium silicate and the graphite. Thus, the non-ortho PCBs recovery rate of the second analysis sample is high, and the accuracy of fractionation into the dioxin group including the non-ortho PCBs, the PCDDs, and the PCDFs and the mono-ortho PCBs is high.

### Comparative Examples 7 to 11

In the dioxin analysis solution preparation apparatus illustrated in Fig. 1 and used in Examples 1 to 16 and Comparative Examples 1 to 6, an adsorbent layer portion of the fractionating layer was, as shown in Table 8, changed to a double-layer stack of an upper layer and a lower layer. Moreover, in Comparative Example 11, no alumina layer was provided.

[Table 8]

**Table 8**

| | Adsorbent layer | | | | | | | Alumina Layer | | | Dioxin Solution |
|---|---|---|---|---|---|---|---|---|---|---|---|
| | Upper Layer | | | Lower Layer | | | Volume Ratio (A:B) between Upper Layer (A) and Lower Layer (B) | Filler | Filling Amount (g) | Filling Height (mm) | |
| | Filler | | Filling Amount (g) | Lower Layer | | Filling Amount (g) | | | | | |
| Comparative Example 7 | Activated Carbon-Containing Silica Gel | Activated Carbon Content: 0.018% by weight | 0.06 | Graphite-Containing Silica Gel | Graphite Content: 12.5% by weight | 0.25 | 1:5 | Alumina | 0.755 | 32.5 | Food Sample Solution A |
| Comparative Example 8 | Activated Carbon-Containing Silica Gel | Activated Carbon Content: 0.018% by weight | 0.06 | Graphite-Containing Silica Gel | Graphite Content: 12.5% by weight | 0.25 | 1:5 | Alumina | 0.755 | 32.5 | Food Sample Solution B |
| Comparative Example 9 | Activated Carbon-Containing Silica Gel | Activated Carbon Content: 0.018% by weight | 0.06 | Graphite-Containing Silica Gel | Graphite Content: 12.5% by weight | 0.25 | 1:5 | Alumina | 0.755 | 32.5 | Environmental Sample Solution D |
| Comparative Example 10 | Activated Carbon-Containing Silica Gel | Activated Carbon Content: 0.018% by weight | 0.06 | Graphite-Containing Silica Gel | Graphite Content: 12.5% by weight | 0.25 | 1:5 | Alumina | 0.755 | 32.5 | Simulant Environmental Sample Solution |
| Comparative Example 11 | Activated Carbon-Containing Silica Gel | Activated Carbon Content: 0.013% by weight | 0.06 | Activated Carbon-Containing Silica Gel | Activate d Carbon Content: 3% by weight | 0.23 | 1:5 | - | - | - | Standard Solution |

Using the preparation apparatus changed as described above, the dioxin analysis sample preparation operation was executed as in Examples 1 to 16 and Comparative Examples 1 to 6. Each of the obtained first analysis sample and the obtained second analysis sample was separately quantitatively analyzed by the HRGC/HRMS method, and the rate of recovery of dioxins and non-DL-PCBs was calculated. Results are shown in Table 9. In Table 9, "-" indicates that no recovery rate was calculated.

### [Table 9]

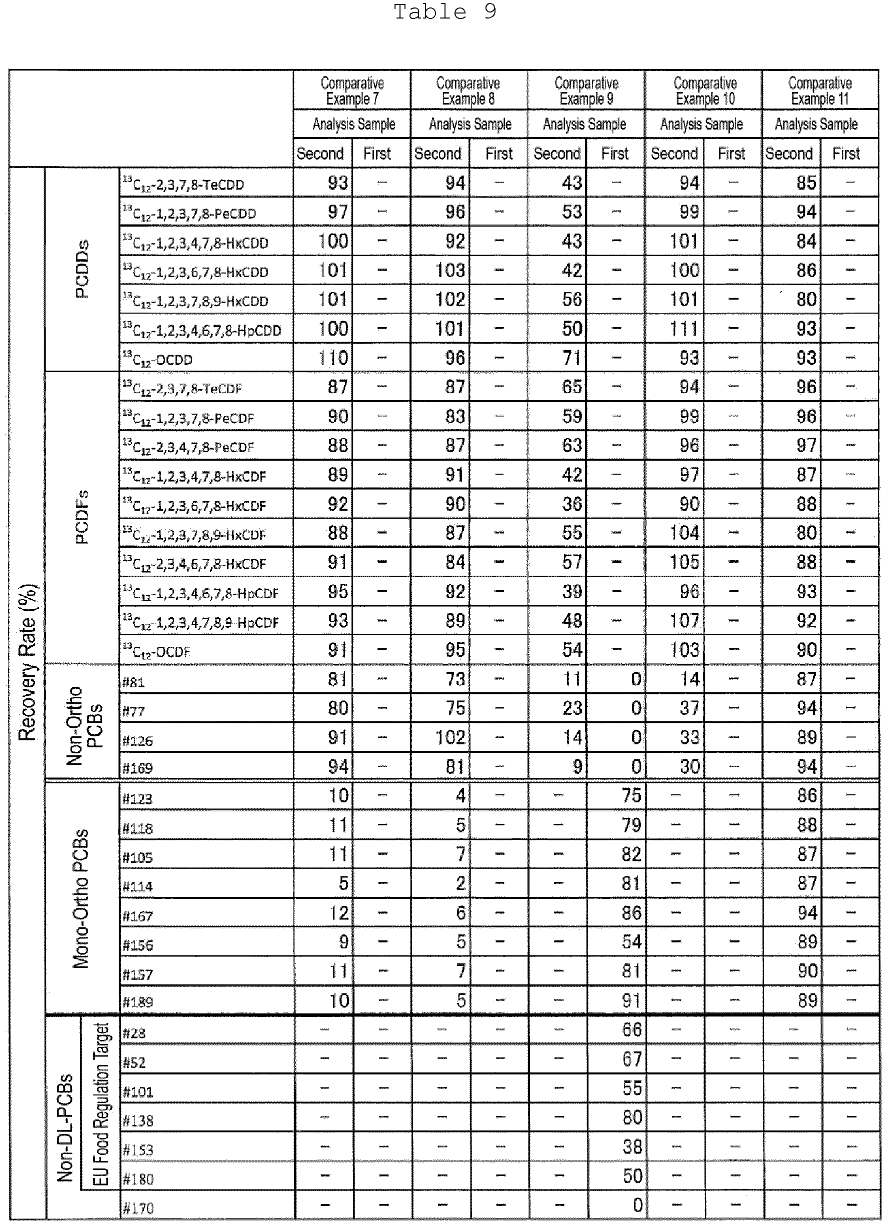

According to Table 9, the fractionating tools of Comparative Examples 7 to 10 with the same adsorbent layer can fractionate the dioxins with a high accuracy in a case where the dioxin solution is a food sample solution. In a case where the dioxin solution is an environmental sample solution containing an aromatic hydrocarbon compound as an impurity component, non-ortho PCBs trapping performance in the adsorbent layer is degraded, and as a result, the dioxin fractionating accuracy is degraded.

### LIST OF REFERENCE NUMERALS

- 200: Fractionating Tool
- 210: Pipe Body
- 220: Purification Layer
- 221: Silver Nitrate Silica Gel Layer
- 222: Sulfuric Silica Gel Layer
- 240: Adsorbent layer
- 250: Alumina Layer

## Claims

1. A method for fractionating dioxins, comprising:
a step of passing an aliphatic hydrocarbon solvent solution containing the dioxins to pass through an adsorbent layer using an adsorbent mixed active magnesium silicate.

2. The method for fractionating the dioxins according to claim 1, wherein
the adsorbent further contains graphite mixed with the active magnesium silicate.

3. The method for fractionating the dioxins according to claim 2, wherein
a content of the graphite in the adsorbent is equal to or lower than 25% by weight.

4. The method for fractionating the dioxins according to claim 2 or 3, wherein
the active magnesium silicate is prepared in such a manner that a mixture of magnesium silicate and graphite is heated to equal to or lower than 650°C.

5. The method for fractionating the dioxins according to any one of claims 1 to 4, wherein
the aliphatic hydrocarbon solvent solution having passed through the adsorbent layer further passes through an alumina layer.

6. The method for fractionating the dioxins according to claim 5, further comprising:
a step of supplying a solvent capable of dissolving the dioxins to the adsorbent layer through which the aliphatic hydrocarbon solvent solution has passed, and then collect the solvent having passed through the adsorbent layer; and
a step of supplying a solvent capable of dissolving the dioxins to the alumina layer through which the aliphatic hydrocarbon solvent solution has passed and then collect the solvent having passed through the alumina layer.

7. A tool for fractionating dioxins contained in a dioxin solution, comprising:
a pipe body opening at both ends; and
an adsorbent layer using an adsorbent mixed active magnesium silicate filled into the pipe body.

8. The tool for fractionating the dioxins according to claim 7, wherein
the adsorbent further contains graphite mixed with the active magnesium silicate.

9. The tool for fractionating the dioxins according to claim 8, wherein
a content of the graphite in the adsorbent is equal to or lower than 25% by weight.

10. The tool for fractionating the dioxins according to any one of claims 7 to 9, wherein:
an alumina is filled into the pipe body.

11. A sample preparation method for dioxin analysis, comprising:
a step of applying a dioxin solution to a purification layer including a silver nitrate silica gel layer and a sulfuric silica gel layer;
a step of supplying an aliphatic hydrocarbon solvent to the purification layer to which the dioxin solution has been applied;
a step of passing the aliphatic hydrocarbon solvent having passed through the purification layer to an adsorbent layer filled with an adsorbent mixed active magnesium silicate;
a step of causing the aliphatic hydrocarbon solvent having passed through the adsorbent layer to pass through an alumina layer;
a step of collecting the solution, as a first fraction eluted from alumina layer, which supplying a solvent capable of dissolving the dioxins to the alumina layer through which the aliphatic hydrocarbon solvent has passed; and then, sequentially,
a step of collecting the solution, as a second fraction eluted from the adsorbent layer, which supplying a solvent capable of dissolving the dioxins to the adsorbent layer through which the aliphatic hydrocarbon solvent has passed.

12. A method for determining dioxins contained in a sample solution, comprising:
a step of separately performing accurate quantitative determination, by a GC/MS or a bioassay, the first fraction and the second fraction prepared according to claim 11.
